# EUROPEAN PATENT SPECIFICATION

(11) **EP 3 510 172 B1**
(45) Date of publication and mention of the grant of the patent: **29.11.2023**
(21) Application number: 17847818.6
(22) Date of filing: 07.09.2017
(51) Int. Cl.: C12Q 1/70

(54) **METHODS OF DETECTING HIV**
VERFAHREN ZUM NACHWEIS VON HIV
MÉTHODES DE DÉTECTION DU VIH

(30) Priority: 07.09.2016 AU 2016903599
(43) Date of publication of application: 17.07.2019
(73) Proprietor: St Vincent's Hospital Sydney Limited, Darlinghurst, NSW 2010 (AU)
(72) Inventor: SUZUKI, Kazuo, Pymble, New South Wales 2073 (AU)
(74) Representative: D Young & Co LLP
(86) International application number: PCT/AU2017/050974
(87) International publication number: WO 2018/045425

(56) References cited:
- WO-A1-2012/013732
- US-A- 6 025 124
- US-A- 6 025 124
- US-A1- 2013 261 137
- US-A1- 2014 370 496
- ADAMS M ET AL: "Limitation of Tat-Associated Transcriptional Processivity in HIV-Infected PBMC", VIROLOGY, ELSEVIER, AMSTERDAM, NL, vol. 257, no. 2, 10 May 1999 (1999-05-10), pages 397-405, XP004439951, ISSN: 0042-6822, DOI: 10.1006/VIRO.1999.9647
- ALEXANDER O PASTERNAK ET AL: "Cell-associated HIV RNA: a dynamic biomarker of viral persistence", RETROVIROLOGY, BIOMED CENTRAL LTD., LONDON, GB, vol. 10, no. 1, 15 April 2013 (2013-04-15) , page 41, XP021145645, ISSN: 1742-4690, DOI: 10.1186/1742-4690-10-41
- BERKHOUT B ET AL: "SECONDARY STRUCTURE OF THE HIV-2 LEADER RNA COMPRISING THE TRNA-PRIMER BINDING SITE", NUCLEIC ACIDS RESEARCH ADVANCE ACCESS, OXFORD UNIVERSITY PRESS, GB, vol. 21, no. 5, 11 March 1993 (1993-03-11) , pages 1171-1178, XP001121818, ISSN: 0305-1048
- SHAN L et al.: "A Novel PCT Assay for Quantification of HIV-1 RNA", Journal of Virology, vol. 87, no. 11, 2013, pages 6521-6525, XP055488620,
- AITKEN SC et al.: "Development and Evaluation of an Affordable Real-Time Qualitative Assay for Determining HIV-1 Virological Failure in Plasma and Dried Blood Spots", Journal of Clinical Microbiology, vol. 51, no. 6, 2013, pages 1899-1905, XP055488622,
- ROUET F et al.: "In-house HIV-1 RNA real-time RT-PCR assays: principle, available tests and usefulness in developing countries", Expert Review of Molecular Diagnostic s, vol. 8, no. 5, 2008, pages 635-650, XP055593352, ISSN: 1473-7159, DOI: 10.1586/14737159.8.5.635
- BERKHOUT B et al.: "Structural features in the HIV-1 repeat region facilitate strand transfer during reverse transcription", RNA, vol. 7, 2001, pages 1097-1114, XP055488624,
- DATABASE Geneseq [Online] 22 October 2015 (2015-10-22), "HIV-2 LTR DNA (H2LTR1) detection Taqman probe P, SEQ ID 55.", XP55810705, retrieved from EBI accession no. GSN:BCD71933 Database accession no. BCD71933

## Description

### RELATED APPLICATION DATA

The present application claim priority from Australian Patent Application No. 2016903599 entitled "Methods of detecting lentivirus" filed 7 September 2016.

### SEQUENCE LISTING

The present application is filed with a Sequence Listing in electronic form.

### FIELD OF THE DISCLOSURE

The present disclosure is based on methods for detecting and quantifying lentivirus (human immunodeficiency virus, HIV-1 or HIV-2) DNA and RNA in a biological sample.

### BACKGROUND

The human immunodeficiency virus (HIV) is a lentivirus that causes HIV infection and overtime is the causative agent of Acquired Immunodeficiency Syndrome (AIDS). HIV belongs to the retroviridae family of viruses, and two types of HIV have been characterised: HIV-1 and HIV-2. HIV-1 is more virulent, and therefore more infective virus and the cause of the majority of infections globally.

HIV is transmitted as single-stranded, positive-sense enveloped RNA virus (ssRNA). The primary target of HIV is CD4⁺ T cells, macrophages and dendritic cells. The HIV virion enters into the target cell by the adsorption of glycoproteins on its surface to receptors on the target cell followed by fusion of the viral envelope with the cell membrane and release of the HIV capsid into the cell. Upon entry into the target cell the nucleocapsid containing the viral genome dissociates, releasing the contents of the virus, including the ssRNA, into the cytoplasm. The viral RNA genome is reverse transcribed into double stranded DNA by a virally encoded reverse transcriptase (RT) enzyme. The resulting viral DNA is then imported into the cell nucleus and integrated into the cellular DNA by a virally encoded integrase.

The integrated HIV DNA is flanked by identical 5' and 3' long terminal repeat (LTR) sequences from which HIV can initiate transcription of the integrated HIV genome. The integrated viral DNA may lie dormant, in the latent stage of HIV infection or the viral DNA may be transcribed, producing new RNA genomes and viral proteins that are packaged and released from the cell as new viral particles.

The primary test for detecting HIV is an enzyme-linked immunosorbent assay (ELISA) to detect HIV-1 antibodies. If a positive test result is received, a Western Blot test is usually conducted to confirm the diagnosis.

Therapy for HIV infection includes combination antiretroviral therapy (cART), which acts to suppress HIV replication. The primary goal of cART is to suppress the plasma viral load to undetectable levels (<50 copies per ml), whilst maintaining function of the immune system and preventing opportunistic infections. Plasma viral load (pVL) monitoring is currently the most important predictor of response to treatment with cART. Levels higher than 200 copies per ml are considered virologic failure. Whilst cART reduces the extent of infection, residual virus forms a viral reservoir, which resides in long-lived resting T cells and tissue-based macrophages. Upon cessation of ART, pVL levels rebound rapidly, usually within a few weeks in the vast majority of patients. There is currently no reliable assay available to monitor and evaluate the treatment outcome of ART for HIV infected patients. While pVL and CD4⁺ cell number still play an important role in patient care, these markers are not sensitive enough to monitor active HIV infection.

Accordingly, there is a need in the art for the development of more sensitive assays that can detect and quantify HIV DNA and RNA, in particular HIV transcription in the latently infected reservoir to guide improvements in anti-retroviral therapy.
US 6 025 124 A discusses a method of HIV detection comprising the amplification of the TAR sequence.

### SUMMARY OF THE DISCLOSURE

The present disclosure is based on methods for detecting and quantifying lentivirus (human immunodeficiency virus, HIV-1 or HIV-2) DNA and RNA in a biological sample. In particular, the present disclosure is based on the finding that a more sensitive detection of lentivirus infection in an HIV-infected subject is obtained when detection of both HIV DNA and HIV RNA is carried out. The inventor has developed PCR based assays which provide more sensitive detection of HIV-1 or HIV-2 DNA and RNA compared to prior art methods. Additionally, the inventor has developed a PCR based method for detection and quantification of HIV DNA and RNA where existing assays fail to detect HIV DNA or RNA.

Current methods of HIV detection and quantitation in diagnostic laboratories rely on detection of HIV plasma Viral Load (VL) (HIV RNA copy number in plasma). This assay is able to quantify HIV RNA copy number in patient's plasma in order to evaluate efficacy of anti-retroviral therapy (ART) treatment of the infected patient. This classical marker of pVL stills play an important role, however this assay is not sensitive enough to adequately identify patients who are receiving optimal anti-retroviral therapy (ART) and thus prone to relapsing.

The present disclosure advantageously provides highly specific and sensitive methods based on amplification of the R region within the long terminal repeats (LTRs) of the lentivirus genome (e.g. HIV genome). The 5' and 3' LTR regions consist of three sub-regions, namely U3, R and U5 and both the 5' and 3' LTRs are present when the virus integrates into the host cell genome. In particular, the inventor has found that prior art strategies which are focussed on targeting either the 3' LTR, *pol* or *gag* are less sensitive compared to R region detection, since the former are only present as a single copy within the viral genome whereas the R region will be present as two copies within the transcribed viral mRNA as well as integrated viral HIV DNA in the human genome. Accordingly, the methods of the present disclosure are based on R region detection within both the RNA and DNA of either HIV-1 or HIV-2 in subjects having, or suspected of having an HIV infection. The present methods thus provide the clinician with a knowledge of the HIV DNA level in the subject as well as the HIV transcription level (RNA) in latently infected reservoir cells (typically CD4⁺ T cells and monocytes/macrophages) in the subject to more accurately guide appropriate treatment with anti-retroviral therapy.

The present disclosure provides a method of detecting human immunodeficiency virus (HIV) in a subject with HIV-1 or HIV-2 or a subject suspected of having an HIV infection (acquired immune deficiency syndrome, AIDS), the method comprising performing PCR amplification of R region nucleic acid in a biological sample obtained from the subject, wherein the amplification comprises forward and reverse primers which hybridise to sequences within the R region of the long terminal repeats (LTRs) of HIV, and subsequently detecting any amplification, wherein detecting amplification is indicative of the presence of HIV in the subject, wherein the amplification will result in both R regions present within the 5' and 3' LTRs to be amplified, and wherein:
(i) the forward primer is a HIV-1 primer consisting of the sequence SEQ ID NO:7, or SEQ ID NO:29; and the reverse primer is a HIV-1 primer consisting of the sequence SEQ ID NO:9, or SEQ ID NO:30; or
(ii) the forward primer is a HIV-2 primer consisting of the sequence SEQ ID NO:14, SEQ ID NO:33, or SEQ ID NO:35; and the reverse primer is a HIV-2 primer consisting of the sequence SEQ ID NO:16, SEQ ID NO:32, SEQ ID NO:34 or SEQ ID NO:36.

Preferably, detecting amplification is performed with a labelled oligonucleotide probe which hybridises to a sequence within the amplified R region sequence.

In one example, the nucleic acid is DNA or reverse-transcribed RNA.

In one example, the PCR amplification is real-time PCR or end-point PCR.

In another example, PCR amplification is quantitative real-time PCR.

In one example, the detection method further comprises a labelled oligonucleotide probe. In another example, the oligonucleotide probe binds to a sequence within the R region of the long terminal repeats (LTRs) of HIV. In another example, the oligonucleotide probe is a hydrolysis probe. In another example, the probe is a TaqMan^{®} probe. In a further example, the oligonucleotide probe is a fluorescently labelled hybridisation probe. In certain examples, the probe may comprise one or more locked nucleic acids.

In one example, the method further comprises:
(i) obtaining an aliquot of the sample wherein the DNA (or reverse transcribed RNA) has been extracted;
(ii) contacting the aliquot with a labelled hydrolysis oligonucleotide probe which hybridises to R region sequence of a long terminal repeat (LTR) of the HIV DNA;
(iii) contacting the aliquot with forward and reverse primers which hybridise to sequences within the R region sequence;
(iv) amplifying the R region sequence by PCR.

The present disclosure also provides a method of quantifying HIV DNA copy number in a biological sample from a subject with HIV, or suspected of having an HIV infection, the method comprising:
(i) amplifying and detecting HIV-R region sequence as described herein; and
(ii) quantifying the amplified HIV-R region sequence by reference to a corresponding HIV plasmid standard to obtain HIV-R region copy number of HIV DNA per volume of sample.

In one example, the amplification is real-time PCR. In another example, the amplification is end-point PCR.

In one example, the method further comprises normalising the HIV DNA copy number against a DNA standard to obtain the HIV DNA copy number per cell(s) in the biological sample.

The present disclosure also provides a method of quantifying HIV DNA copy number in a biological sample from a subject with HIV, or suspected of having an HIV infection by normalising to a standard, the method comprising:
(i) amplifying and detecting HIV-R region sequence as described herein;
(ii) quantifying by quantitative PCR, the HIV-R region copy number per volume of DNA in the sample by reference to a corresponding HIV standard;
(iii) quantifying an endogenous housekeeping gene by quantitative PCR using a corresponding housekeeping standard to obtain the copy number for the endogenous gene expressed as copies of housekeeping gene per volume of DNA present in the sample;
(iv) dividing the obtained copy number by the number of copies of the endogenous gene in a cell to derive the cell number per volume of DNA in the sample; and
(v) normalising the HIV DNA copy number by calculating the HIV-R region DNA copy number per cell by dividing the value obtained in (i) with the value obtained in (iii) to obtain the HIV R region DNA copy number (copies/cell) in the biological sample.

In one example, the DNA standard is actin.

In one example, the quantitative PCR is real-time PCR.

In a further example, the HIV DNA copy number is expressed as the HIV R region copies/10⁶ cells.

Alternatively, quantification of HIV DNA copy number in the sample may be obtained from measuring the absorbance of DNA in the sample.

The present disclosure also provides a method for quantifying HIV DNA copy number in a biological sample from a subject with HIV, or suspected of having an HIV infection, the method comprising:
(i) amplifying and detecting HIV-R region sequence as described herein;
(ii) quantifying the mass of DNA per volume (w/v) in the sample by measuring absorbance of DNA in the sample;
(iii) calculating the cell number per volume of DNA in the sample based on DNA absorbance; and
(v) normalising the HIV DNA copy number by calculating the HIV-R region DNA copy number per cell by dividing the value obtained in (ii) with the value obtained in (iv) to obtain the HIV R region DNA copy number (copies/cell) in the biological sample.

In one example, the method of quantifying the mass of DNA per volume in the sample alternatively comprises the addition of a DNA intercalating dye and measuring the fluorescence emitted from the dye. In one example, the DNA intercalating dye is selected from SyBr Green I, Syto-9, Syto-10-14, Syto-16, Syto-21, Syto-24, Syto-29, YoYo-1, YoYo-3 and ToTo-1.

In one example, the HIV DNA copy number is expressed as the HIV R region copies/10⁶ cells.

In one example, the methods described herein may further comprise:
(i) obtaining an aliquot of the sample wherein the DNA has been extracted;
(ii) contacting the aliquot with a labelled hydrolysis oligonucleotide probe which hybridises to R region sequence of a long terminal repeat (LTR) of the HIV DNA;
(iii) contacting the aliquot with forward and reverse primers which hybridise to sequences within the R region sequence;
(iv) amplifying the R region sequence by PCR;
(v) extrapolating the signal obtained from the labelled oligonucleotide to a standard curve obtained by corresponding amplifications of serial dilutions of an HIV standard to derive the HIV-R region DNA copy number per volume of DNA in the aliquot.

In one example, the PCR is real-time PCR or end-point PCR.

In one example, the oligonucleotide is a hydrolysis oligonucleotide probe (e.g. TAqMan^{®} probe). In one example, the oligonucleotide is a fluorescently labelled hybridisation probe.

In one example, the forward and reverse primers bind to R region sequences upstream and downstream respectively of the R region sequence to which the oligonucleotide binds.

In certain examples, PCR amplification is performed by end-point PCR. In one example, the forward or reverse primer is labelled. In one example, the forward or reverse primer is labelled (e.g with biotin). In one example, the oligonucleotide probe is labelled with digoxigenin (Dig).

In one example, the labelled probe comprises or consists of the sequence according to SEQ ID NO:4, SEQ ID NO:12, SEQ ID NO:37, SEQ ID NO:41; SEQ ID NO:45, SEQ ID NO:47, SEQ ID NO:49, or SEQ ID NO:51.

The methods for quantifying DNA described herein can be used to monitor anti-retroviral therapy (ART) being administered to an HIV positive subject.

Accordingly, the present disclosure also provides a method for monitoring anti-retroviral therapy (ART) being administered to an HIV positive subject, comprising quantifying the HIV DNA copy number as described herein over at least two time points and comparing the difference in HIV DNA copy number between the at least two time points wherein a decrease in the HIV DNA copy number indicates the subject is receiving optimal/effective ART.

Biological samples may be obtained over multiple time points over the life of the subject, including but not limited to three, four, five, six, eight, ten, twelve, fifteen, twenty, twenty-five, thirty, thirty-five, forty etc. times. In another example, the period between the at least two time points is days, weeks or months. In another example, the period between the at least two time points is 1 week, 2 weeks, 1 month, 3 months, four months, six months, eight months, or twelve months.

In another example, a decrease of at least 50%, at least 45%, at least 40%, at least 35%, at least 30%, at least 25%, at least 20%, at least 15%, at least 12%, at least 10%, at least 8%, or at least 5% between time points indicates that the subject is receiving optimal/effective ART.

In another example, an HIV DNA copy number of about 800 or less copies of HIV DNA per 10⁶ cells indicates the subject is receiving optimal/effective ART. In another example, a HIV DNA copy number of about 80 or less, or about 8 or less copies of HIV R region DNA per 10⁶ cells in the biological sample indicates the subject is receiving optimal/effective ART.

The present disclosure also provides a method for quantifying HIV RNA copy number in a biological sample from a subject with HIV, or suspected of having an HIV infection, the method comprising:
(i) amplifying and detecting HIV-R region sequence as described herein on reverse-transcribed HIV RNA R region sequence; and
(ii) quantifying the amplified HIV-R region sequence by reference to a corresponding HIV plasmid standard to obtain the copy number of HIV RNA per volume of sample.

In one example, the amplification is real-time PCR or end-point PCR.

In one example, the method further comprises normalising the HIV RNA copy number against a RNA standard to obtain the HIV RNA copy number per cell(s) in the biological sample.

The present disclosure also provides a method for quantifying HIV RNA copy number (i.e. transcriptional activity) in a biological sample from a subject with HIV, or suspected of having an HIV infection by normalising to a standard, the method comprising:
(i) amplifying and detecting HIV-R region sequence as described herein on reverse-transcribed HIV RNA R region sequence;
(ii) quantifying by quantitative PCR, the HIV-R region copy number per volume of RNA in the sample by reference to a corresponding HIV standard;
(iii) quantifying an endogenous housekeeping gene by quantitative PCR using a corresponding housekeeping standard to obtain the copy number for the endogenous gene expressed as copies of housekeeping gene per volume of RNA present in the sample;
(iv) dividing the obtained copy number by the number of copies of the endogenous gene in a cell to derive the cell number per volume of RNA in the sample; and
(v) normalising the HIV RNA copy number by calculating the HIV-R region RNA copy number per cell by dividing the value obtained in (i) with the value obtained in (iii) to obtain the HIV R region RNA copy number (copies/cell) in the biological sample.

In one example, the RNA standard is GAPDH.

In one example, the method further comprises obtaining a biological sample from the subject and preparing RNA from the sample.

In one example, the HIV RNA copy number is expressed as the HIV R region copies/10⁶ cells.

In another example, the housekeeping standard is amplified by real-time PCR. In yet another example, the housekeeping standard is the same as the endogenous gene. Methods of generating a standard curve based on amplifying serial dilutions of a standard will be known to persons skilled in the art. In example, the housekeeping standard is serially diluted to provide 1, 20, 200, 2000, 20,000, 2 ×10⁵, and 2 ×10⁶ copies/µl.

Alternatively, the method of quantifying RNA in the sample is based on measuring the absorbance of RNA in the sample.

The present disclosure also provides a method for quantifying HIV RNA copy number in a biological sample from a subject with HIV, or suspected of having an HIV infection, the method comprising:
(i) amplifying and detecting HIV-R region sequence as described herein on reverse-transcribed HIV RNA R region sequence;
(ii) quantifying the mass of RNA per volume (w/v) in the sample by measuring absorbance of RNA in the sample;
(iii) calculating the cell number per volume of RNA in the sample based on RNA absorbance; and
(iv) normalising the HIV RNA copy number by calculating the HIV-R region RNA copy number per cell by dividing the value obtained in (ii) with the value obtained in (iv) in order to obtain the HIV R region RNA copy number (copies/cell) in the biological sample.

In one example, quantifying the mass of RNA per volume in the sample comprises an RNA intercalating dye and measuring fluorescence omitted from the dye.

In one example, the RNA copy number is expressed as the HIV-R region copies/10⁶ cells.

In a further example, the method of quantifying the mass of RNA per volume in the sample comprises the addition of an RNA intercalating dye and measuring the fluorescence emitted from the dye. In one example, the RNA intercalating dye is selected from SyBr Green II, Syto RNA select green-fluorescent and ToTo-1.

Persons skilled in the art will appreciate that methods of quantifying the mass of DNA or RNA per volume in the sample will be known in the art. For example, such methods include, but are not limited to Qubit (Thermo Fisher), Nanodrop (ThermoFisher), and QuantideX (Asuragen).

In one example, the HIV-R region is quantified by:
(i) obtaining an aliquot of the sample wherein the RNA has been extracted;
(ii) contacting the aliquot with a labelled hydrolysis oligonucleotide probe which hybridises to the R region sequence of a long terminal repeat (LTR) of the reverse transcribed HIV RNA (cDNA);
(iii) further contacting the aliquot with forward and reverse primers which hybridise to sequences within the R region of the reverse transcribed HIV RNA (cDNA);
(iv) amplifying the R region sequence by PCR;
(v) extrapolating the signal obtained from the labelled oligonucleotide to a standard curve obtained by corresponding amplifications of serial dilutions of an HIV standard to derive the HIV-R region RNA copy number per volume of RNA in the aliquot.

In one example, the forward and reverse primers bind to R region sequences upstream and downstream respectively of the R region sequence to which the oligonucleotide binds.

In one example, the housekeeping standard is amplified by real-time PCR. In another example, the housekeeping standard is the same as the endogenous gene.

The present disclosure also provides a method for assessing the effectiveness of anti-retroviral therapy (ART) administered to an HIV positive subject, the method comprising:
(i) quantifying the HIV-R region DNA copy number as described herein;
(ii) quantifying the HIV-R region RNA copy number as described herein;
(iii) determining a normalised HIV RNA copy number in a sample obtained from the subject by dividing the value obtained in step (i) with that obtained in step (ii); and
(iv) comparing the normalised HIV RNA copy number value to one or more previous normalised values obtained from the same subject;
wherein a decrease in the normalised HIV RNA copy number indicates that the subject is receiving optimal/effective ART.

In one example, quantifying the HIV-R region DNA or RNA copy number is according to a method described herein or known in the art.

The HIV DNA copy number or HIV RNA copy number as described herein may be expressed as the number of copies per 1,000,000 cells (10⁶) i.e. copies/10⁶ cells. In one example, the HIV RNA copy number is expressed as a value for any fixed number of cells, including but not limited to 1.5 × 10⁶, 2 × 10⁶, 2.5 × 10⁶ or 3 × 10⁶ cells.

In one example, the volume is expressed as µl or ml.

The methods described above are preferably performed in a polymerase chain reaction (PCR) tube.

The biological sample may be a population of cells selected from blood or tissue or any other biological fluid in which lentivirus-infected cells are present. The biological sample may be whole blood, plasma or peripheral blood mononuclear cells (PBMCs) or sorted samples of enriched CD4⁺ T cells and monocytes/macrophages or separated samples using magnetic beads to enrich the cell population subset (e.g. CD4⁺ T cells and monocytes/macrophages). The biological sample may also include DNA or RNA derived from whole blood or PBMCs. The PBMCs may be separated from whole blood using Ficoll or Ficoll-Paque. In one example, the biological sample is obtained from a subject. In one example, the subject is a human or primate.

It will be appreciated by persons skilled in the art that the R region of sequence within both the 5' and 3' LTRs of the lentivirus/HIV nucleic acid will be amplified by the foregoing methods. In one example, the R region sequence amplified is that contained within the sequence consisting of the sequence set forth in SEQ ID NO:1 or SEQ ID NO:2. In one example, the R region sequence consists of the sequence set forth in SEQ ID NO:1 or SEQ ID NO:2.

In another example, the housekeeping standard may be selected from the group consisting of glyceraldehyde 3-phosphate dehydrogenase (GAPDH), beta actin (β-actin), beta-2 microglobulin (B2M), peptidylprolyl isomerase A (PPIA), eukaryotic translation elongation factor 1 gamma (EEF1γ), succinate dehydrogenase complex subunit A (SDHA), hydroxymethyl-bilane synthase (HMBS), 18s ribosomal RNA (18s rRNA) and phosphoglycerate kinase 1 (PGK 1).

In one example, the housekeeping gene and housekeeping standard for HIV DNA is β-actin. In another example, the housekeeping gene and housekeeping standard for HIV RNA is GAPDH.

In one example, cell number in the sample is determined by measuring the DNA mass after DNA extraction. In another example, DNA mass is estimated by absorbance of spectrophotometer at 260nm. In one example, cell number in the sample is determined by measuring the RNA mass after RNA extraction. In another example, RNA amount is estimated by absorbance of spectrophotometer at 260nm.

In one example, cell number in the sample is determined by measuring the DNA mass after DNA extraction. In another example, DNA mass is estimated by measuring fluorescence emitted from DNA intercalating dye; In one example, cell number in the sample is determined by measuring the RNA mass after RNA extraction. In another example, RNA amount is estimated by measuring fluorescence emitted from RNA intercalating dye.

In one example, the labelled or hydrolysis oligonucleotide is a TAQMAN^{®} probe. TAQMAN^{®} probes comprise a fluorophore covalently attached to the 5' end of the oligonucleotide probe and a quencher at the 3' end for real time PCR quantification. In one example, the fluorophore is selected from the group consisting of hydroxycoumarin, methoxycoumarin, Alexa fluor, aminocoumarin, Cy2, Alexa fluor 488, 430, 532, 546, 555, 594, 633, 660, 680 FITC, TRITC, PE, LC Cyan500, FAM, TET, JOE, Yakima Yellow, HEX, Cy3, TAMARA, ROX, Texas Red, LC Red610, LC Red640, Cy5, Cy5.5, Cy7 and IRD700. In one example the quencher is selected from the group consisting of BHQ-1, BHQ-2, IBRQ, IBFQ. In one example the probe is a double quencher. In another example, the probe is a double quencher comprising a ZEN^{™} internal quencher combined with the IBRQ, IBFQ quencher.

In one example, according to any method described herein, the oligonucleotide binds to a sequence comprising or consisting of the sequence 5' TAAGCAGTGGGTTCCCT 3' (SEQ ID NO:3) or a sequence at least 90% identical thereto. In a further example, the oligonucleotide binds to a sequence at least 92%, at least 94%, at least 95%, at least 96%, at least 97%, at least 98%, at least 99% or at least 99.5% identical to the sequence set forth in SEQ ID NO:3.

In a further example the oligonucleotide comprises or consists of the sequence 5' AGGGAACCCACTGCTTA 3' (SEQ ID NO:4). In a further example, the oligonucleotide comprises or consists of the sequence X-AGGGAACCCACTGCTTA-Z (SEQ ID NO:5) wherein X is a reporter molecule, Z is a quencher molecule and the sequence optionally contains at least one locked nucleic acid (LNA). In a further example, X is FAM (carboxyfluorescein) and Z is BHQ-1. In a further example, the oligonucleotide comprises between 1 and 6 LNAs, between 2 and 5 LNAs or between 2 and 4 LNAs. In a further example, the oligonucleotide comprises or consists of the sequence FAM-AGG_{LNA}GA_{LNA}AC_{LNA}CCAC_{LNA}TG_{LNA}CTTA-BHQ-1 (SEQ ID NO:6) wherein X is a reporter molecule, Z is a quencher molecule and LNA is a locked nucleic acid. For the sake of clarity, a locked nucleic acid is referred to herein as N_{LNA} wherein N is the indicated A, T, C or G nucleobase.

The person skilled in the art will appreciate that the LNA modification is not restricted to the aforementioned positions and the number of location of LNA modifications will be ascertained in order to improve specificity and reduce back ground of the real-time PCR assay.

The forward primer may consist of the sequence 5' GAGCCTGGGAGCTCTCTG 3' (SEQ ID NO:7) and the reverse primer mayconsist of the sequence 5' ACTCAAGGCAAGCTTTATTGAGGC 3' (SEQ ID NO:9).

In another example, the oligonucleotide binds to a sequence comprising or consisting of the sequence 5' GCCTGGGTGTTCCCTGCTAGACTCT 3' (SEQ ID NO:11) or a sequence at least 90% identical thereto. In a further example, the oligonucleotide binds to a sequence at least 92%, at least 94%, at least 95%, at least 96%, at least 97%, at least 98%, at least 99% or at least 99.5% identical to the sequence set forth in SEQ ID NO:11.

In another example, the oligonucleotide comprises or consists of the sequence 5' AGAGTCTAGCAGGGAACACCCAGGC 3' (SEQ ID NO:12). In a further example, the oligonucleotide comprises or consists of the sequence X-GCCTGGGTGTTCCCTGCTAGACTCT -Z (SEQ ID NO:13) wherein X is a reporter molecule, Z is a quencher molecule. In a further example, X is FAM (carboxyfluorescein) and Z is BHQ-1.

The present disclosure provides a composition for amplifying HIV-1 nucleic acid, comprising a labelled oligonucleotide probe comprising or consisting of a combination of probe, forward and reverse primer selected from a combination of one or more of the following:
(i) oligonucleotide probe: SEQ ID NO:4, SEQ ID NO:5, SEQ ID NO:6, SEQ ID NO:45, SEQ ID NO:46, SEQ ID NO:47, or SEQ ID NO:48;
(ii) forward primer: SEQ ID NO:7 or SEQ ID NO:29; and
(iii) reverse primer: SEQ ID NO:9 or SEQ ID NO:30.

The present disclosure also provides a composition for amplifying HIV-2 nucleic acid, comprising a labelled oligonucleotide comprising or consisting of a combination of probe, forward and reverse primer selected from a combination of one or more of the following:
(i) oligonucleotide probe: SEQ ID NO:12, SEQ ID NO:13, SEQ ID NO: 37, SEQ ID NO: 38, SEQ ID NO:39, SEQ ID NO:40; SEQ ID:41, SEQ ID:42, SEQ ID:43, SEQ ID:44, SEQ ID NO:49, SEQ ID NO:50, SEQ ID NO:51, SEQ ID NO:52 or SEQ ID NO:53;
(ii) forward primer: SEQ ID NO:14, SEQ ID NO:33, or SEQ ID NO:35;
(iii) reverse primer: SEQ ID NO:16, SEQ ID NO:32, SEQ ID NO:34 or SEQ ID NO:36.

The present disclosure also provides a kit for detecting HIV-1, comprising the composition as described herein, together with suitable reagents and instructions for detecting and quantifying HIV-1 according to the methods described herein.

The present disclosure also provides a kit for detecting HIV-2 comprising the composition as described herein, together with suitable reagents and instructions for detecting and quantifying HIV-2 according to the methods described herein.

### BRIEF DESCRIPTION OF THE DRAWINGS

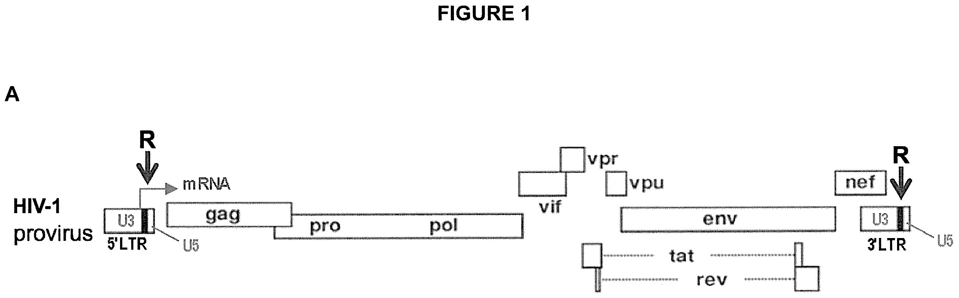

**Figure 1** shows the genomic structure of HIV-1.

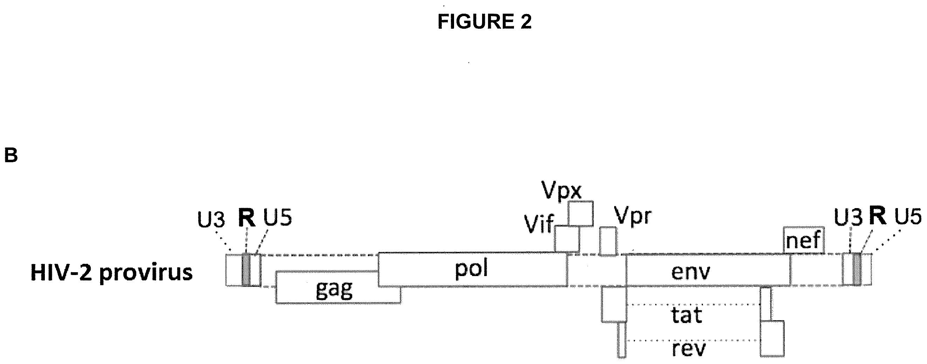

**Figure 2** shows the genomic structure of HIV-2.

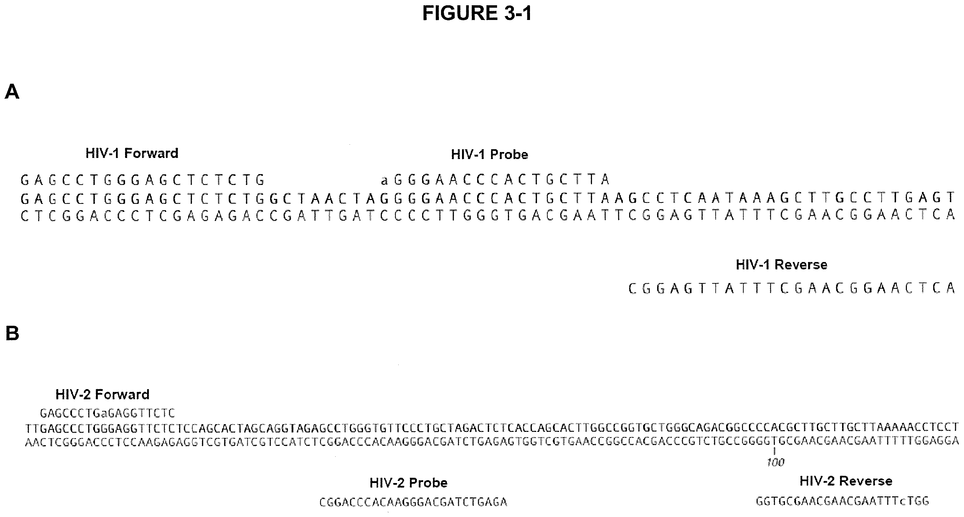

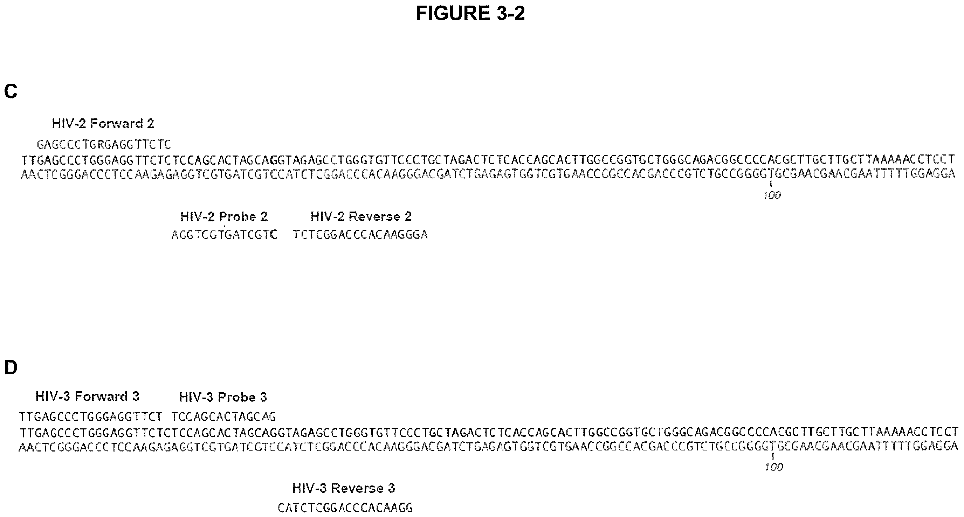

**Figure 3** shows the location of the PCR primers and oligonucleotide probe to the R region sequence of HIV-1 (**A**). The R region of the HIV-1 sequence is 95 bases long. **B, C** and **D** shows the location of the PCR primers and oligonucleotide probe combinations exemplified herein to the R region sequence of HIV-2. The R region of the HIV-2 sequence is 174 bases long. Locked nucleic acid modifications were introduced into some oligonucleotide probes to increase the annealing temperature of the probe.

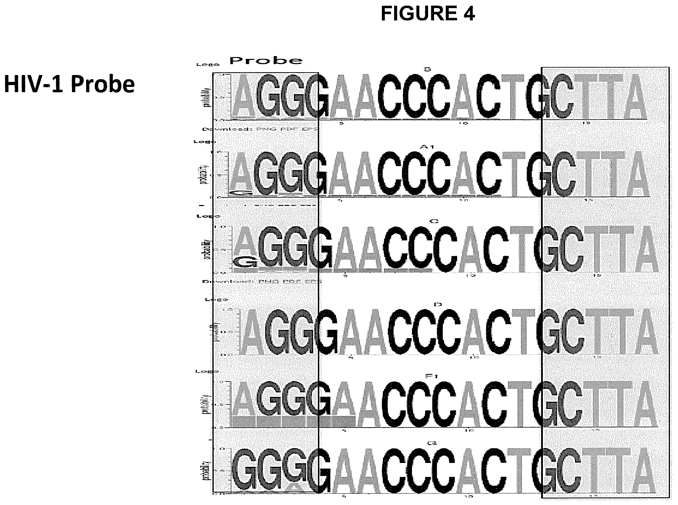

**Figure 4** shows the alignment of the oligonucleotide probe (HIV-1 probe 1) to the HIV-1 subtype sequences. Shading indicates where sequences are conserved between HIV-1 subtypes to provide efficient PCR. HIV sequences were obtained from the Los Alamos National Laboratory- HIV Sequence Consortium 2013 database (HIV Sequence Compendium 2013 Foley B, et al. Published by Theoretical Biology and Biophysics Group, Los Alamos Laboratory NM, LA-UR 13-26007).

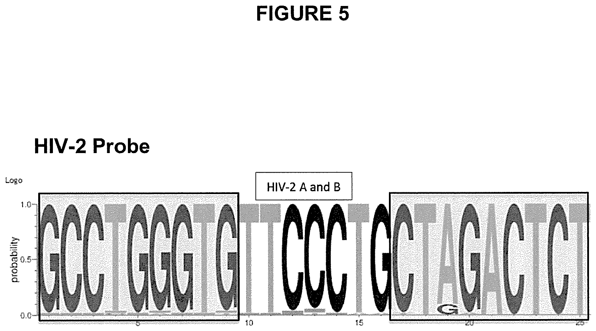

**Figure 5** shows the sequence within HIV-2 subtypes that the HIV-2 probe 1 binds.

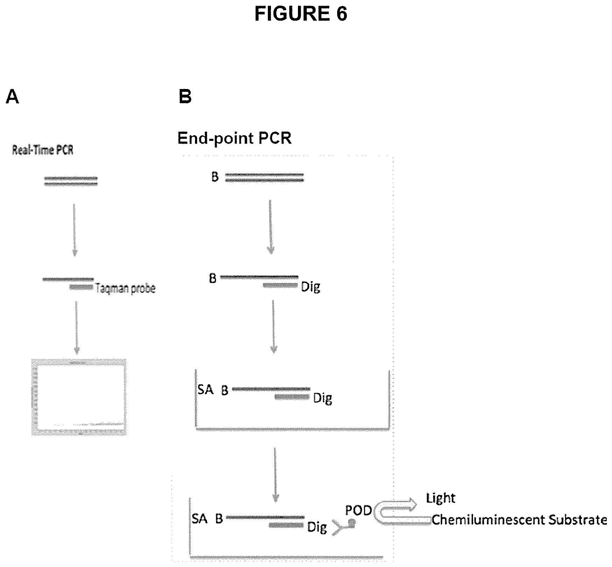

**Figure 6** shows a schematic of real-time PCR assay (A) and end-point PCR assay (B). The real-time PCR assay uses a Taqman probe and unlabelled primer sets. The end-point PCR assay in this embodiment uses a biotin labelled reverse primer and unlabelled forward primer and a digoxigenin (Dig) labelled probe. A peroxidase labelled antibody against Dig is added, followed by a chemiluminescent reaction to provide an output in relative light units (RLU).

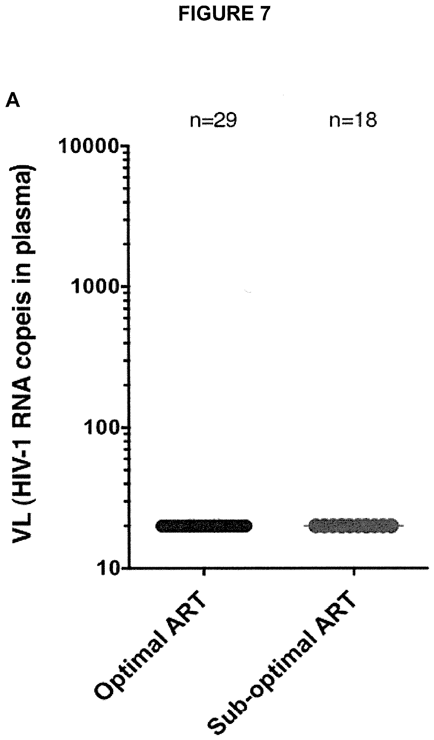

**Figure 7** shows that the plasma viral load assay is not able to discriminate optimal ART subjects from sub-optimal ART subjects. All 46 subjects (Sub-optimal ART and optimal ART groups) showed supressed VL (VL <20 copies/mL) at the time of collection of blood.

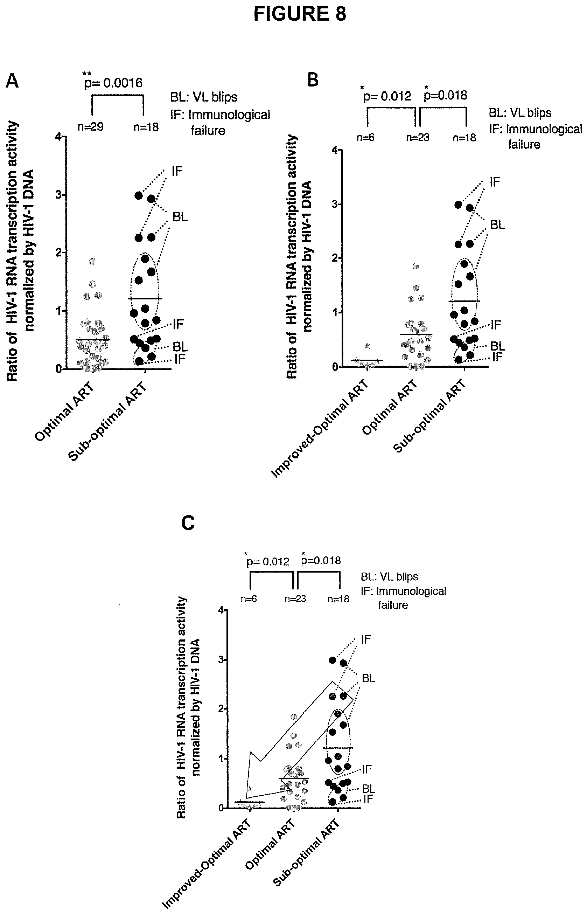

**Figure 8** shows intracellular RNA amplification and quantification according to the present methods using a GAPDH standard, discriminated the "sub-optimal ART" (n=18) and "Optimal ART" (n=29) subject groups and the difference was statistically significant (**A**). Further analysis based on HIV-1 DNA level defines "Improved-Optimal ART" group, where HIV-1 DNA level is less than 800 HIV-1 DNA per 10⁶ cells (**B**). The difference in HIV-1 RNA transcriptional activity of "Improved-Optimal ART" (n=6) and "Optimal ART" (n=23) was statistically significant (**B** and C). "Blip" experienced and "immunological failure" patients are classified as Sub-optimal ART subjects, where Blip is at least one episode of transient high pVL (less than 200 copies/mL) within 6 months. Immunological failure is defined as a pVL of <20 copies/ml and CD4⁺ T cell count <350 counts/µl, while Optimal ART subjects were showing suppressed VL for more than 6 months (VL <20 copies/mL).

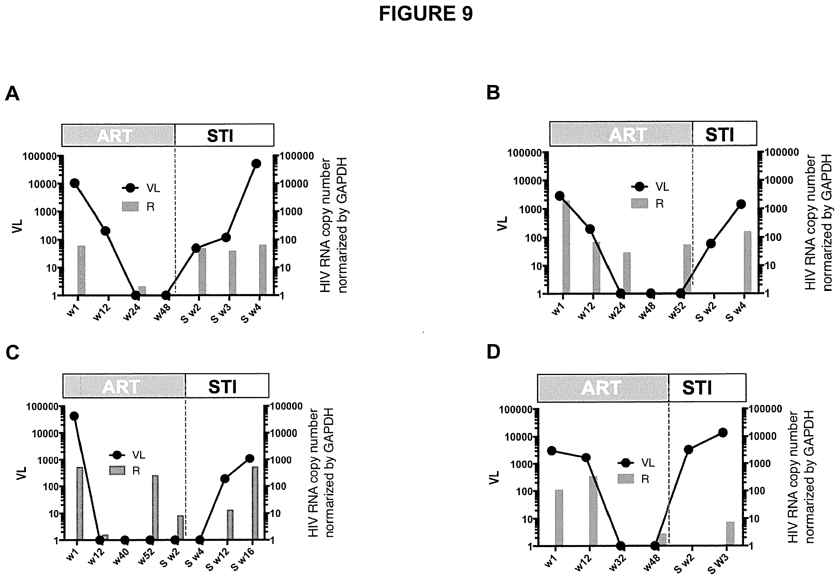

**Figure 9** shows a typical VL data for treatment success patients and rebounded HIV-1 VL after stopping ART treatment. Four treatment naive patients were treated with ART for one year. The level of plasma Viral Load (pVL, black line with circles) in four patents was under the detection limit during 24 week long treatment (**A**, **B**, and **D**) and during 12 week long treatment (C) The pVL level was kept under the detection level until week 52 (one year) of ART treatment. Despite undetectable pVL in the four patents, low level of intracellular HIV-1 transcriptional activities were detected (grey column). After stopping therapy there was rapid elevation of the VL in four patients and elevated intracellular HIV-1 transcriptional activity was accompanied with increased VL. ART= anti-retroviral therapy. STI= Stricture Treatment Interruption (STI: stopping ART).

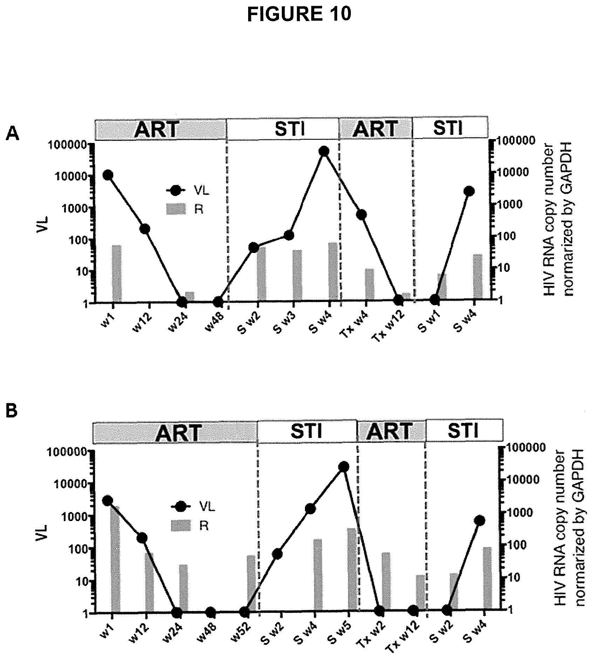

**Figure 10** Subjects in Figure 9 went into the second phase of the ART treatment. During this second phase ART period, VL was suppressed under detection level, however, consistent detection of intracellular HIV-1 transcriptional activity even with VL under detection level (grey column). After stopping the second phase of ART treatment, a quick rebound VL was observed in both patients (A and B). ART= anti-retroviral therapy. STI= Stricture Treatment Interruption (STI: stopping ART).

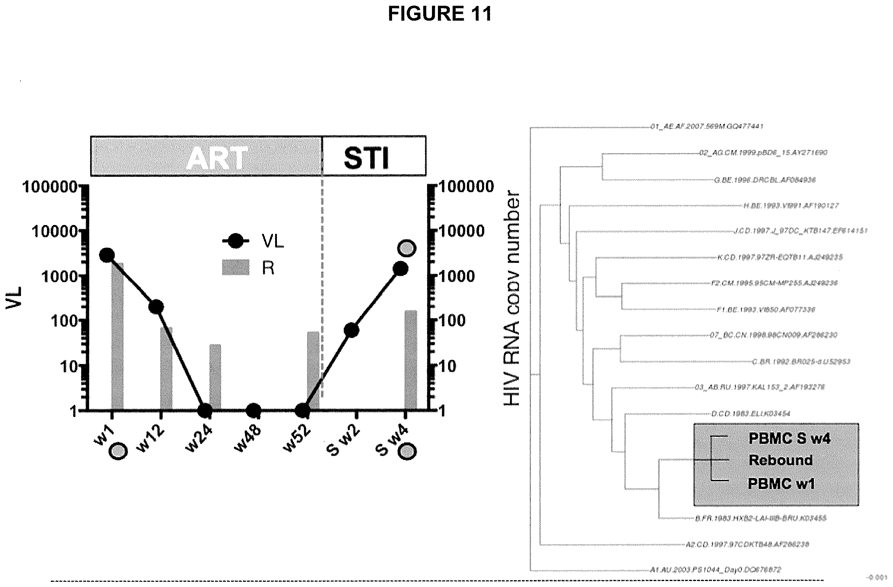

**Figure 11** To investigate whether the increase in transcriptional activity was derived from cells in the latently infected reservoir, intracellular RNA at w1 (ART) and at week 4 after stopping ART (sw4), as shown as grey circles on the horizontal axis. Rebounded HIV-1 plasma RNA at week 16 post-ART (upper grey circle) were collected and analysed by Sanger sequencing. Data was analysed using a neighbour joining clustering method on a web-based HIV database (http://www.hiv.lanl.gov/content/sequence/HIV/HIVTools.html). Samples isolated from the same subjects clustered together (grey rectangle box) suggesting that the latent viral reservoir has persistent HIV transcriptional activity that contributes to the plasma viral load rebound.

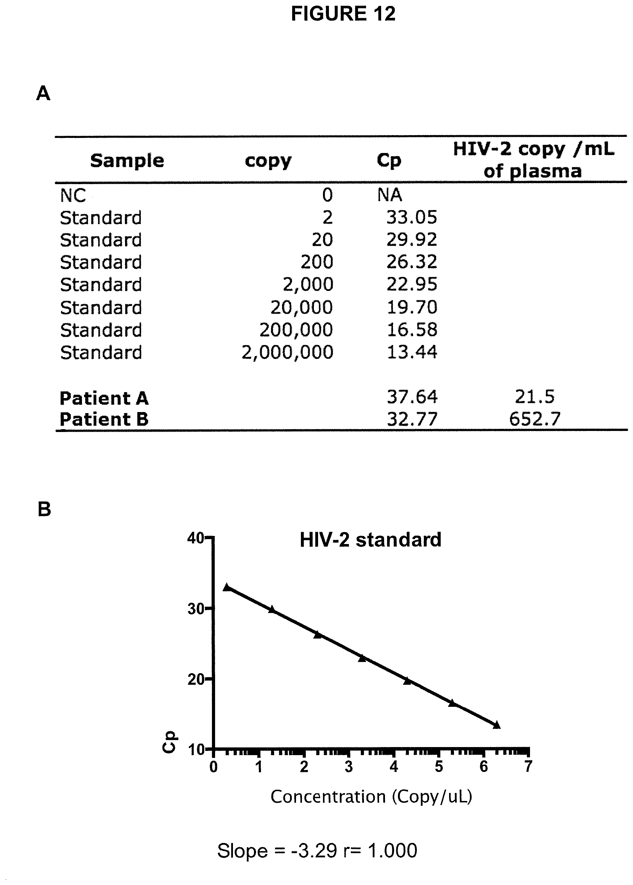

**Figure 12** shows TaqMan probe based PCR analysis and quantitation for HIV-2 RNA obtained from two subjects' plasma samples. (**A**) shows the analysis of HIV-2 copy/ml of plasma in the samples along with HIV-2 standards. HIV-2 RNA copy number was estimated from the Cp value generated from 7 standards. (**B**) shows a standard analysis for the HIV-2 assay. Cp values generated by the standards are indicated by open circles.

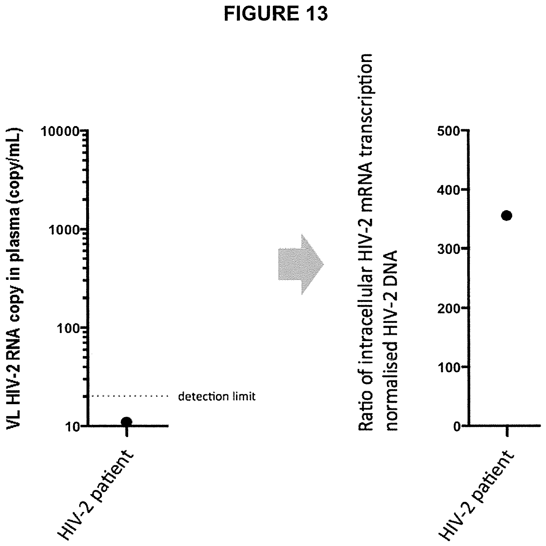

**Figure 13** shows intracellular HIV-2 transcript analysis conducted in a subject with plasma viral load less than the detection limit. Despite a negative plasma viral load detection, HIV-2 intracellular transcripts (HIV-2 RNA) were detected when normalised to integrated HIV-2 DNA copy number.

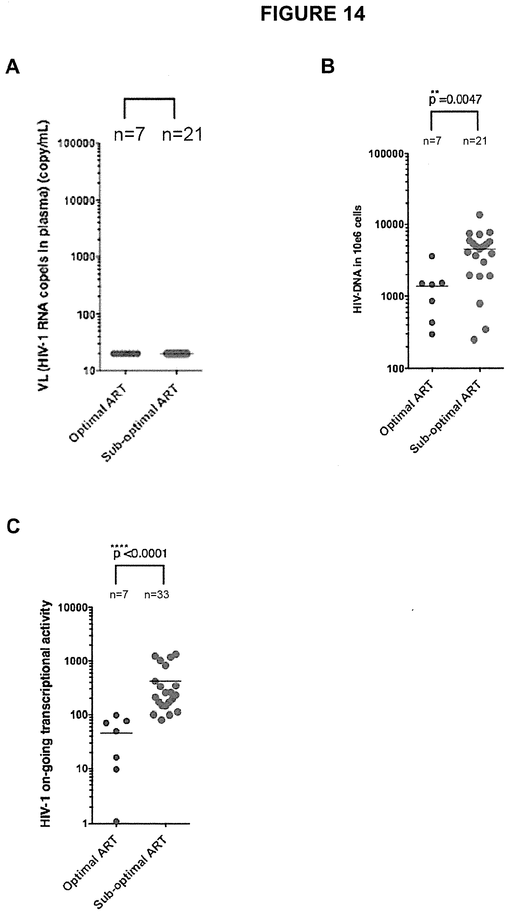

**Figure 14** shows analysis of 28 subjects for plasma viral load (**A**). as shown in (**A**) there was no detectable HIV-1 in the plasma of the subjects. (**B**) shows the quantification of HIV-1 DNA in optimal (n=7) and sub-optimal (n=21) ART subjects. (**C**) shows HIV-1 transcriptional activity (RNA).

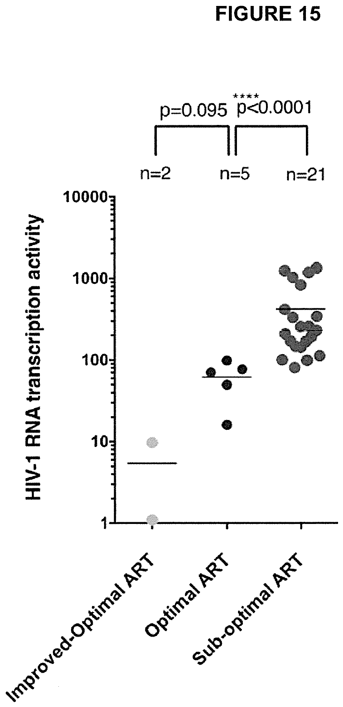

**Figure 15** shows HIV-1 transcriptional activity (RNA) in improved-optimal ART, optimal Art and sub-optimal ART subjects.

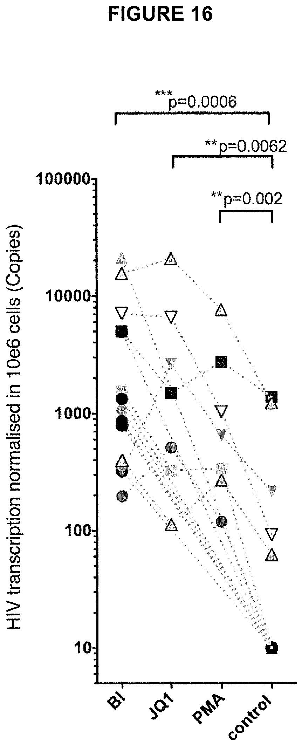

**Figure 16** shows HIV transcription normalised to GAPDH of activated HIV-1 in latent HIV-1 infected cells.

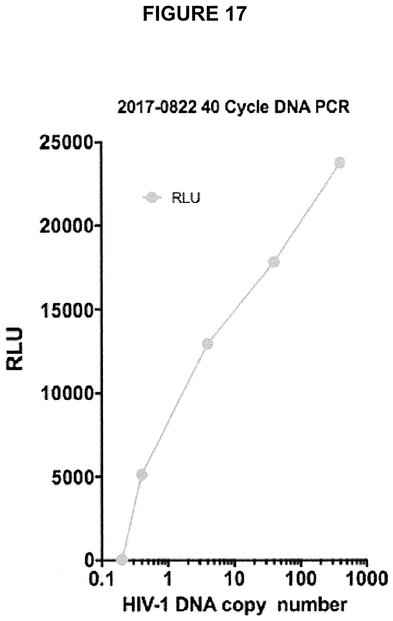

**Figure 17** shows standard curve for 40 cycle end-point amplification of HIV-1 DNA standard.

### TABLE OF SEQUENCES

The following Table summarises the sequences referred to in this disclosure.

| **SEQ ID No** | **Sequence** | **Description** |
|---|---|---|
| 1 | 5' GTCTCTCTGGTTAGACCAGATCTGAGCCTGGGAGCT CTCTGGCTAACTAGGGAACCCACTGCTTAAGCCTCAA TAAAGCTTGCCTTGAGTGCTTC 3' | Sequence of R region of HIV-1 |
| 2 | 5’ TTGAGCCCTGGGAGGTTCTCTCCAGCACTAGCAGGT AGAGCCTGGGTGTTCCCTGCTAGACTCTCACCAGCA CTTGGCCGGTGCTGGGCAGACGGCCCCACGCTTGCT TGCTTAAAAACCTCCT 3’ | Sequence of R region of HIV-2 |
| 3 | 5' TAAGCAGTGGGTTCCCT 3' | Sequence within the R region that the HIV-1 probe oligo binds |
| 4 | 5' AGGGAACCCACTGCTTA 3' | Sequence of the HIV-1 R region probe oligo |
| 5 | 5' X-AGGGAACCCACTGCTTA-Z 3' | Sequence of the HIV-1 R region probe oligo |
| 6 | 5' FAM- AGG_{LNA}GA_{LNA}AC_{LNA}CCAC_{LNA}TG_{LNA}CTTA-BHQ-1 3' | Sequence of the HIV-1 R region probe oligo |
| 7 | 5' GAGCCTGGGAGCTCTCTG 3' | HIV-1 forward R region primer |
| 8 | 5' CAGAGAGCTCCCAGGCTC 3' | Sequence within the R region of HIV-1 that the forward primer binds |
| 9 | 5' ACTCAAGGCAAGCTTTATTGAGGC 3' | HIV-1 reverse R region primer |
| 10 | 5' GCCTCAATAAAGCTTGCCTTGAGT 3' | Sequence within the R region of HIV-1 that the reverse primer binds |
| 11 | 5' GCCTGGGTGTTCCCTGCTAGACTCT 3' | Sequence within the R region that the HIV-2 oligo binds |
| 12 | 5' AGAGTCTAGCAGGGAACACCCAGGC 3' | Sequence of the HIV-2 R region probe oligo |
| 13 | 5' X- AGAGTCTAGCAGGGAACACCCAGGC -Z 3' | Sequence of the HIV-2 R region probe oligo |
| 14 | 5' GAGCCCTGAGAGGTTCTC 3' | HIV-2 forward R region primer |
| 15 | 5' GAGAACCTCCCAGGGCTC 3' | Sequence within the R region of HIV-2 that the forward primer binds |
| 16 | 5' GGTCTTTAAGCAAGCAAGCGTGG 3' | HIV-2 reverse R region primer |
| 17 | 5' FAM AAGGTCGGAGTCAACGGATTTGGTCGT BHQ-1 3' | GAPDH probe oligo |
| 18 | 5' GGCAACAATATCCACTTTACCAG 3' | GAPDH forward primer |
| 19 | 5' TCGACAGTCAGCCGCATCTT 3' | GAPDH reverse primer |
| 20 | 5' FAM ATGCCCTCCCCCATGCCATCCTGCG BHQ-1 3' | β-actin probe oligo |
| 21 | 5' TCACCCACACTGTGCCCATCTACGA 3' | β-actin forward primer |
| 22 | 5' CAGCGGAACCGCTCATTGCCAATGG 3' | β-actin reverse primer |
| 23 | 5' CCAAAGAAGACAAGATATCCTTGA 3' | 3' LTR forward primer |
| 24 | 5' TTGAGGCTTAAGCAGTGG 3' | 3' LTR reverse primer |
| 25 | 5′ FAM-TTAGACCAGATCTGAGCCTGGGAGCTCTC- BHQ-1 3′ | 3' LTR probe oligo |
| 26 | 5' AGTGGGGGGACATCAAGCAGCCATGCAAAT 3' | gag forward primer |
| 27 | 5' TACTAGTAGTTCCTGCTATGTCACTTCC 3' | gag reverse primer |
| 28 | 5′-FAM- ATCLNAALNAATGLNAAGGAAGLNACTGLNAC -BHQ-1 3′ | gag probe oligo |
| 29 | 5' GAGCCYGGGAGCTCYCTG 3' Y refers to a T/C mix wherein the proportion of T is 95% and the proportion of C is 5%. | HIV-1 forward R region primer (HIV-1 For 2) |
| 30 | 5' ACTCAAGGCAAGCTTTATTGAG 3' | HIV-1 reverse R region primer (HIV-1 Rev 2) |
| 31 | 5' GAGCCCTGRGAGGTTCTC 3' R refers to a G/A mix wherein the proportion of G is 90% and the proportion of A is 10% | HIV-2 forward R region primer (HIV-2 For 1) |
| 32 | 5' GGTYTTTAAGCAAGCAAGCGTGG 3' Y refers to a T/C mix wherein the proportion of T is 10% and the proportion of C is 90%. | HIV-2 reverse R region primer (HIV-2 Rev 1) |
| 33 | 5'-GAGCCCTGRGAGGTTCTC 3' R refers to a G/A mix wherein the proportion of G is 90% and the proportion of A is 10% | HIV-2 forward R region primer (HIV-2 For 2) |
| 34 | 5' AGGGAACACCCAGGCTCT 3' | HIV-2 reverse R region primer (HIV-2 Rev 2) |
| 35 | 5' TTGAGCCCTGGGAGGTTCT 3' | HIV-2 forward R region primer (HIV 2 For 3) |
| 36 | 5' GGAACACCCAGGCTCTAC 3' | HIV-2 reverse R region primer (HIV-2 Rev 3) |
| 37 | 5'- CTGCTAGTGCTGGA -3' | Sequence of the HIV-2 R region probe oligo (HIV-2 Pro 2) |
| 38 | 5' X- CTGCTAGTGCTGGA -Z 3' | Sequence of the HIV-2 R region probe oligo (HIV-2 Pro 2) |
| 39 | 5' FAM-CTGCTAGTGCTGGA BHQ-1 3' | Sequence of the HIV-2 R region probe oligo (HIV-2 Pro 2) |
| 40 | 5'- FAM- CT_{LNA}GC_{LNA}TA_{LNA}GT_{LNA}G_{LNA}CTGG_{LNA}A BHQ-1 3' | Sequence of the HIV-2 R region probe oligo (HIV-2 Pro 2) |
| 41 | 5'- TCCAGCACTAGCAG -3' | Sequence of the HIV-2 R region probe oligo (HIV-2 Pro 3) |
| 42 | 5' X- TCCAGCACTAGCAG -Z 3' | Sequence of the HIV-2 R region probe oligo (HIV-2 Pro 3) |
| 43 | 5' FAM- TCCAGCACTAGCAG BHQ-1 3' | Sequence of the HIV-2 R region probe oligo (HIV-2 Pro 3) |
| 44 | 5′- FAM- TCLNACALNAGCLNA ACLNAT ALNAGCLNAAG BHQ-1 3′ | Sequence of the HIV-2 R region probe oligo (HIV-2 Pro 3) |
| 45 | 5'- TTTTTTTTTTTTTTTTGGAACCCACTGCTTA -3' | Sequence of the HIV-1 R region probe oligo (HIV-1 Pro 2) |
| 46 | 5' X- GGAACCCACTGCTTA -Z 3' | Sequence of the HIV-1 R region probe oligo (HIV-1 Pro 2) |
| 47 | 5' AGGGAACCCACTGCT 3' | Sequence of the HIV-1 R region probe oligo (HIV-1 Pro 3) |
| 48 | 5'- X- AGGGAACCCACTGCT -Z 3' | Sequence of the HIV-1 R region probe oligo (HIV-1 Pro 3) |
| 49 | 5' TTTTTTTTTTTTTTTTCCAGCACTAGCAGGT 3' | Sequence of the HIV-2 R region probe oligo (HIV-2 Pro 4) |
| 50 | 5'- X- TCCAGCACTAGCAGGT -Z 3' | Sequence of the HIV-2 R region probe oligo (HIV-2 Pro 4) |
| 51 | 5' CTCCAGCACTAGCAG 3' | Sequence of the HIV-2 R region probe oligo (HIV-2 Pro 5) |
| 52 | 5'- X- CTCCAGCACTAGCAG -Z 3' | Sequence of the HIV-2 R region probe oligo (HIV-2 Pro 5) |
| 53 | 5' CT_{LNA}CCA_{LNA}G_{LNA}CA_{LNA}CTA_{LNA}GC_{LNA}AG 3' | Sequence of the HIV-2 R region probe oligo (HIV-2 Pro-5) |

### DETAILED DESCRIPTION

### General

Throughout this specification, unless specifically stated otherwise or the context requires otherwise, reference to a single step, composition of matter, group of steps or group of compositions of matter shall be taken to encompass one and a plurality (i.e. one or more) of those steps, compositions of matter, groups of steps or groups of compositions of matter.

The present disclosure is not to be limited in scope by the specific examples described herein, which are intended for the purpose of exemplification only. The invention is as defined in the appended claims.

Any example of the present disclosure herein shall be taken to apply *mutatis mutandis* to any other example of the disclosure unless specifically stated otherwise.

Unless specifically defined otherwise, all technical and scientific terms used herein shall be taken to have the same meaning as commonly understood by one of ordinary skill in the art (for example, in cell culture, molecular genetics, immunology, immunohistochemistry, protein chemistry, and biochemistry).

Unless otherwise indicated, the recombinant protein, cell culture, and immunological techniques utilized in the present disclosure are standard procedures, well known to those skilled in the art. Such techniques are described and explained throughout the literature in sources such as, J. Perbal, A Practical Guide to Molecular Cloning, John Wiley and Sons (1984), J. Sambrook et al. Molecular Cloning: A Laboratory Manual, Cold Spring Harbor Laboratory Press (1989), T.A. Brown (editor), Essential Molecular Biology: A Practical Approach, Volumes 1 and 2, IRL Press (1991), D.M. Glover and B.D. Hames (editors), DNA Cloning: A Practical Approach, Volumes 1-4, IRL Press (1995 and 1996), and F.M. Ausubel et al. (editors), Current Protocols in Molecular Biology, Greene Pub. Associates and Wiley-Interscience (1988, including all updates until present), Ed Harlow and David Lane (editors) Antibodies: A Laboratory Manual, Cold Spring Harbor Laboratory, (1988), and J.E. Coligan et al. (editors) Current Protocols in Immunology, John Wiley & Sons (including all updates until present).

The term "and/or", e.g., "X and/or Y" shall be understood to mean either "X and Y" or "X or Y" and shall be taken to provide explicit support for both meanings or for either meaning.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Reference to the singular forms "a", "an" and "the" is also understood to imply the inclusion of plural forms unless the context dictates otherwise.

Any discussion of documents, acts, materials, devices, articles or the like which has been included in the present specification is not to be taken as an admission that any or all of these matters form part of the prior art base or were common general knowledge in the field relevant to the present disclosure as it existed before the priority date of each claim of this application.

### Selected Definitions

The term "lentivirus" as used herein refers to a genus of viruses of the Retroviridae family. The genome comprises two copies of a positive sense ssRNA inside a conical capsid. Examples of lentiviruses include human (HIV), simian (SIV) and feline (FIV).

The term "HIV" or "human immunodeficiency virus" as used herein refers to HIV-1 or HIV-2 including the various subtypes. The subtypes of HIV-1 include A, B (the most dominant form), C, D, E, F, G, H, J and K. The subtypes of HIV-2 include A, B, C F or G. HIV-1 and HIV-2 are now classified into groups (e.g. HIV-1 M, N, O and P group) corresponding to phylogenetically associated groups or clades. The structures of HIV-1 and HIV-2 are provided in Figure 1.

The term "hydrolysis oligonucleotide" or "probe" as used herein refers to a dual labelled oligonucleotide in which the 5' end is labelled with a fluorescent reporter molecule while the 3' end is labelled with a quencher molecule. The sequence of the probe is specific for a region of interest (i.e. the R region in Figure 1) in the amplified target molecule. Typically the probe length is between 20-30 bases. The probe is designed so that the length of the sequence places the 5' fluorophore and the 3' quencher in close enough proximity so as to suppress fluorescence. During an amplification reaction, when extension reaches the bound hydrolysis probe, the 5'-3' exonuclease activity of the Taq polymerase degrades the probe. Cleavage of the probe separates the fluorescent reporter molecule from the rest of the probe allowing the reporter molecule to fluoresce. With subsequent PCR cycles, the amount of fluorescent reporter released, and hence fluorescence, increases cumulatively. An example of a hydrolysis oligonucleotide is a TaqMan^{®} probe.

The term "identity" and grammatical variations thereof, mean that two or more referenced entities are the same. Thus, where two sequences are identical, they have the same amino acid sequence, at least within the referenced region or portion. Where two nucleic acid sequences are identical, they have the same polynucleotide sequence, at least within the referenced region or portion. The identity can be over a defined area (region or domain) of the sequence. The % identity of a polynucleotide is determined by GAP (Needleman and Wunsch, J. Mol Biol. 48: 444-453.1970) analysis (GCG program) with a gap creation penalty=5, and a gap extension penalty=0.3. Unless stated otherwise, the query sequence is at least 45 nucleotides in length, and the GAP analysis aligns the two sequences over a region of at least 45 nucleotides. Preferably, the query sequence is at least 100 nucleotides in length, and the GAP analysis aligns the two sequences over a region of at least 100 nucleotides. Most preferably, the two sequences are aligned over their entire length.

The term "viral load or VL" as used herein is intended to refer to a measure of the number of viral particles (i.e. HIV-1 or HIV-2) or amount of viral genetic material present in an organism, typically the number of viral particles per volume of plasma.

The term "anti-retroviral therapy" or "ART" as used herein refers to treatment of an HIV positive subject with a combination of several anti-viral medicines which are used to slow the rate at which HIV multiples in the body.

The term "optimal/effective ART" as used herein refers to subjects which have a suppressed plasma viral load (pVL) for greater than 6 months. By "suppressed viral load" it is meant under limit of detection according to pVL.

The term "primer" as used herein refers to an oligonucleotide which is capable of acting as a point of initiation of synthesis when placed under conditions in which synthesis of primer extension product which is complementary to a nucleic acid strand (template) is induced.

The term "sub-optimal/sub-effective ART" as used herein refers to subjects who have experienced at least one blip (BL) or who have immunological failure (IL). A blip refers to a transient viral increase where the viral load rises to between 50 and 100 copies/ml (or about <200 copies/mL). Sub-optimal ART subjects are those that exhibit one or more blips in 6 months. Immunological failure is defined as a pVL of <20 copies/ml and CD4⁺ T cell count <350 counts/µl. The term "Improved Optimal ART" as used herein refers to subjects who have a DNA copy number of less than 800 DNA per 10⁶ cells especially within Optimal ART group patients.

The term "real-time PCR" as used herein refers to any methodology where the amplification of DNA, or reverse transcribed RNA is monitored in real time during the PCR and not at its end as in conventional PCR. Typically, the terms "real-time PCR" and "quantitative PCR" are used interchangeably. Methods for performing real-time PCR are known in the art from resources as described herein. Kits for performing real-time PCR using TaqMan probes are available from commercial supplies e.g. Thermofisher or Applied Biosystems.

The term "Cp" or "crossing point" as used herein refers to the cycle during real-time PCR at which fluorescence from amplification exceeds the background fluorescence. For example, a lower Cp correlates with a higher target expression level in the sample.

The term "RNA" as used herein is also intended to include mRNA.

As used herein, the terms "treat," "treating," "treatment" and grammatical variations thereof mean subjecting an individual subject to a protocol, regimen, process or remedy, in which it is desired to obtain a physiologic response or outcome in that subject. Since every treated subject may not respond to a particular treatment protocol, regimen, process or remedy, treating does not require that the desired physiologic response or outcome be achieved in each and every subject or subject population. Accordingly, a given subject or subject population may fail to respond or respond inadequately to treatment.

The term "binds to" means that the oligonucleotide reacts or associates more frequently, more rapidly, with greater duration and/or with greater affinity with a particular nucleic acid sequence that it does with alternative nucleic acid sequences.

The term "hybridises" as used herein refers to the formation of a double stranded nucleic acid from complementary single stranded nucleic acids. The hybridisation may occur between two nucleic acid stands perfectly matched or substantially matched with some mismatches. The complementarity for hydridisation may depend on hybridisation conditions, particularly temperature. The detailed conditions for hybridisation can be found in Molecular Cloning, A Laboratory Manual, Cold Spring Harbor Laboratory Press, Cold Spring Harbor NY (2001) and MLM Anderson, Nucleic acid hybridisation, Springer-Verlag, New York, NY (1999).

The term "locked nucleic acid" as referred to herein refers to a modified DNA nucleotide in which the deoxyribose moiety of a LNA nucleotide has been modified with an extra bridge connecting the 2' oxygen and 4' carbon.

The term "PBMC" as used herein is intended to refer to any peripheral blood cell having a round nucleus such as T cells, B Cells, NK cells and monocytes. PBMCs also contain progenitor cells. They are typically separated from whole blood using Ficoll or Ficoll-Paque.

### Detection of HIV-1 or HIV-2 and differentiation of HIV-1 and HIV-2 in subjects

Most HIV infected patients are under antiretroviral therapy (ART). ART treatment is able to significantly and rapidly reduce plasma viral load (pVL) (HIV RNA copy number in plasma) to levels below the limit of detection. ART treatment has markedly improved both morbidity and mortality associated with HIV infection however, ART does not lead to a cure. Despite prolonged treatment with ART, HIV persists as integrated HIV DNA in many cell types forming long-lived cellular reservoirs. Upon cessation of ART, pVL levels rebound rapidly, usually within a few weeks in the vast majority of subjects. There is currently no reliable assay available to monitor and evaluate the treatment outcome for ART for HIV (HIV-1 or HIV-2) infected patients. Typically, both pVL and CD4+ T cell counts are used to monitor ART treatment for HIV infected patients. In most of the successfully treated subjects, pVL values are below the limit of detection and CD4+ cell numbers are usually kept at high levels. Although these classical markers still play an important role, they are not sensitive enough to monitor active HIV infection.

Assays based on detecting HIV activity, including proviral DNA, HIV integrated DNA, 2 LTR circles and ultrasensitive viral load in plasma have resulted in lack of sensitivity and/or the requirement for a large volume of specimen.

Currently, detection of HIV in a subject starts with a first serological based screening assay ("HIV-1/2 antigen/antibody combination immunoassay") in which a biological sample, usually blood is tested for HIV antibodies and p24 antigen. If the sample is positive, a second confirmation assay will be performed with the "HIV-1/HIV-2 antibody differentiation immunoassay". Such tests include Western Blot analysis, multisport analysis or Geenius^{™} supplemental assay. This test is used to distinguish between HIV-1 and HIV-2 in the subject. The test has good sensitivity but not good specificity and therefore in some instances, further testing of HIV positive samples and subjects with indeterminate HIV-1/2 status will be done using a nucleic acid test (NAT).

An HIV-1 DNA confirmation assay was developed by Roche (Amplicor HIV-1 DNA PCR Test). However this assay has now been discontinued and HIV diagnostic algorithm is changing from HIV-1 DNA detection to HIV-1 RNA detection using large automated machinery (Cobas 6800/8800 system, M2000, and Panther system). The methodology is based on simultaneous amplification and detection of two separate regions of the HIV-1 genome for quantification of HIV-1 RNA. The machine is intended for processing large numbers of samples, however most HIV reference laboratories do not have large numbers of the samples for confirmation test requests. Moreover, it takes at least eight hours before a result can be obtained and that does not take into account the time associated with transporting the samples to a facility which has the machinery. In many HIV-1 diagnostic laboratories, plasma viral load RNA assay is now used as an alternative HIV-1 conformation assay. However, there are some risks in using HIV-1 plasma VL RNA assay as the HIV-1 conformation assay. In an early HIV-1 infection stage the inventor could not detect plasma VL RNA in plasma, since HIV-1 plasma RNA levels are not elevated at early HIV-1 infection stage. Plasma LV is elevated 2-3 weeks later after HIV-1 infection. Therefore plasma VL HIV-1 RNA assay is not perfectly suitable as an HIV-1 confirmation assay.

There is no HIV-1 DNA assay for confirmation at this moment as the trend has shifted towards the HIV-1 RNA assay. Moreover, the cost of the machine and the cost of testing make the Cobas 6800/8800 system prohibitively expensive for hospital laboratories.

Although the HIV-1 RNA viral load test is sensitive and reliable, there is still a need in the art for HIV-1 DNA detection due to the increasing prevalence of HIV-2. Furthermore, there is a need for more sensitive methodologies to confirm HIV status for samples which are considered to be indeterminate HIV-1/2 and HIV positive samples" by second serology based supplemental confirmation tests.

At present there is no HIV-2 DNA confirmation assay available. The methods described herein provide for the detection and quantification of HIV-1 and HIV-2 DNA that can be carried out in a much shorter time frame and at considerably lower cost and without the need to transport samples to an appropriate facility for testing.

Due to current shifts in the HIV diagnostic algorithm (where the focus has shifted to HIV plasma viral load RNA analysis), there is still a strong need for confirmation assays that can distinguish between HIV-1 and HIV-2 infection in a subject. Such assays are particularly important where a subject is assessed as being "indeterminate HIV-1/2" and "HIV positive" by second serology based supplemental confirmation tests.

The methods of detecting HIV-1 or HIV-2 described herein are based on targeting the R region within the long terminal repeats (LTRs) of the lentivirus. In order to develop the assay, the inventor needed to overcome a number of technical difficulties. First, the length of the R region is short, about 95-175 bases long (see SEQ ID Nos:1 and 2 herein). Typically, a PCR primer length for forward and reverse primers are between 18-25 bases long and the probe length about 20-30 bases long. Therefore, only a limited sequence was available in which the oligonucleotide and primers could bind. A further issue is the potential for primer-dimer formation to occur. Additionally, the primers and oligonucleotide probe must be designed so that they provide reliable and sensitive detection across all subtypes of HIV (HIV-1 or HIV-2). This is illustrated for HIV-1 in for example Figure 1A and Figure 2A and B. Figure 2A shows the alignment of the forward and reverse primer with relatively conserved regions across HIV-1 A, B, C, D, F and G subtypes. Figure 2B shows the alignment of the oligonucleotide probe across HIV-1 subtypes A, B, C, D, F and G. The inventor found that good alignment at the 3' end of the forward primer and perfectly matched 5' end of the reverse primer was necessary to achieve efficient PCR analysis. The inventor also found that the oligonucleotide probe needed to be perfectly matched at the 3' and 5' ends of the probe sequence with the HIV-1 sequence to achieve efficient PCR. The inventor was able to successfully overcome the constraints of a short sequence and multiple subtype variation in sequence to design a quantitative real time - PCR assay that could detect the R region of HIV lentivirus.

### Detecting HIV in a subject

The present disclosure provides a method of detecting human immunodeficiency (HIV) in a subject with HIV-1 or HIV-2 or a subject suspected of having an HIV infection (AIDS), the method comprising performing PCR amplification of nucleic acid in a biological sample obtained from the subject, wherein the amplification comprises primers which hybridise to sequences within the R region of the long terminal repeats (LTRs) of HIV, and subsequently detecting any amplification, wherein detecting amplification is indicative of the presence of HIV in the subject.

Since two copies of the R region are present in the HIV genome, the amplification will result in both R regions present within the 5' and 3' LTRs to be amplified. As disclosed herein,
(i) the forward primer is a HIV-1 primer consisting of the sequence SEQ ID NO:7, or SEQ ID NO:29; and the reverse primer is a HIV-1 primer consisting of the sequence SEQ ID NO:9, or SEQ ID NO:30; or
(ii) the forward primer is a HIV-2 primer consisting of the sequence SEQ ID NO:14, SEQ ID NO:33, or SEQ ID NO:35; and the reverse primer is a HIV-2 primer consisting of the sequence SEQ ID NO:16, SEQ ID NO:32, SEQ ID NO:34 or SEQ ID NO:36.

The PCR amplification may be performed using conventional PCR or quantitative PCR (RT-PCR). In one example, the PCR is performed using real-time PCR.

In one example, the method further comprises a probe. The probe may be a labeled hydrolysis oligonucleotide. In a further example, the probe is a TaqMan^{®} probe. The probe may also be a fluorescently labelled hybridization probe.

In one example, the detection method further comprises:
(i) contacting the nucleic acid in the sample with a labeled hydrolysis oligonucleotide which binds within an R region sequence of the long terminal repeats (LTRs) of HIV;
(ii) further contacting the nucleic acid with forward and reverse primers which hybridise to sequences within the R region sequence;
(iv) amplifying the R region sequence; and
(v) detecting amplification;
wherein amplification is detected by a signal emitted from the labeled oligonucleotide indicating the presence of HIV in the subject.

Preferably the forward and reverse primers bind to R region sequences upstream and downstream respectively of the R region sequence to which the oligonucleotide binds.

In one example, the method further comprises obtaining a biological sample from the subject. In a further example, the biological sample is whole blood, plasma or peripheral blood mononuclear cells (PBMCs). In another example, DNA is extracted from the biological sample.

In another example, the HIV nucleic acid is DNA or RNA. In another example, the method detects integrated HIV. In another example, the amplification is by quantitative PCR. In another example, the amplification is by real-time PCR (RT-PCR). In another example, the hydrolysis oligonucleotide is a TaqMan^{®} probe.

### Quantifying HIV DNA

The present disclosure also provides methods for quantifying HIV DNA copy number in a biological sample. Thus, present disclosure provides a method for quantifying HIV DNA copy number in a biological sample from a subject with HIV, or suspected of having an HIV infection, the method comprising:
(i) detecting the HIV-R region sequence; and
(ii) quantifying the HIV-R region copy number in the sample by reference to an HIV standard, wherein the copy number is expressed as copies of HIV-R region per volume of DNA present in the sample.

In one example, detecting HIV-R region sequence is performed as described herein or known in the art.

In one example, the method further comprises:
(iii) quantifying an endogenous housekeeping gene by quantitative PCR using a corresponding housekeeping standard to obtain the copy number for the endogenous gene expressed as copies of housekeeping gene per volume of DNA present in the sample;
(iv) dividing the obtained copy number by the number of copies of the endogenous gene in a cell to derive the cell number per volume of DNA in the sample; and
(v) normalising the HIV DNA copy number by calculating the HIV-R region DNA copy number per cell by dividing the value obtained in (i) with the value obtained in (iii) to obtain the DNA copy number (copies/cell) in the biological sample.

By "corresponding" it meant that the housekeeping standard is the same as the endogenous housekeeping gene.

Preferably, quantification of HIV-R region DNA and the endogenous housekeeping gene is performed using aliquots of the same sample obtained from the subject. In a particular example, it is performed on aliquots of a sample from the subject in which the DNA has been extracted.

In one example, the HIV-R region is quantified by:
(i) obtaining an aliquot of the sample wherein the DNA has been extracted;
(ii) contacting the aliquot with a labelled oligonucleotide which binds within the R region sequence of a long terminal repeat (LTR) of the HIV DNA;
(iii) further contacting the aliquot with forward and reverse primers which hybridise to sequences within the R region sequence;
(iv) amplifying the R region sequence by quantitative PCR;
(v) extrapolating the signal obtained from the labelled oligonucleotide to a standard curve obtained by corresponding amplifications of serial dilutions of an HIV standard to derive the HIV-R region DNA copy number per volume of DNA in the aliquot.

In another example, the endogenous housekeeping gene is quantified by:
(i) obtaining an aliquot of the sample containing extracted DNA;
(ii) contacting the housekeeping gene in the aliquot with a labelled oligonucleotide which binds to a sequence within the housekeeping gene;
(iii) further contacting the aliquot with forward and reverse primers which hybridise to sequences upstream and downstream respectively of the housekeeping gene sequence to which the oligonucleotide binds;
(iv) amplifying the housekeeping gene sequence using quantitative PCR; and
(v) extrapolating the signal obtained from the labelled oligonucleotide to a standard curve obtained by corresponding amplifications of serial dilutions of the standard to derive the endogenous housekeeping gene copy number per volume of DNA in the aliquot.

In one example, the aliquots used for amplifying HIV R region and the endogenous gene are obtained from the same sample, preferably from DNA extracted from the same sample. In one example, the oligonucleotide is an hydrolysis oligonucleotide.

It will be appreciated by persons skilled in the art that any known housekeeping gene is suitable for use in the present methods. In one example, the housekeeping gene is beta actin. In another example, the housekeeping standard is serially diluted to provide 1, 20, 200, 2000, 20,000, 2 ×10⁵, and 2 ×10⁶ copies/µl.

The cell number per volume of DNA in the biological sample is obtained by quantifying an endogenous housekeeping gene by real-time PCR using a standard that is the same as the endogenous housekeeping gene to obtain the copy number for the endogenous gene which is expressed as a value of X copies/µl of DNA in the sample. The cell number may then be estimated from the actin copy number by dividing by 200 (assuming there are 200 copies of beta actin per cell) to provide a value of X cells/ µl of DNA in the sample. The person skilled in the art will appreciate that depending on the housekeeping gene used, the value of 200 for beta actin would need to be replaced with a different value corresponding to the number of copies of that gene in the cell.

In another example, the HIV standard is a plasmid standard containing a single copy of the HIV genome.

In another example, the HIV standard is serially diluted to provide 0, 4, 40, 400, 4 × 103, 4 x104, 4 × 10⁵ and 4 ×10⁶ copies/µl.

Alternatively, the HIV DNA copy number in the sample can be obtained by replacing the use of a housekeeping gene with calculations based on absorbance of DNA. Therefore, the use of a housekeeping gene standard would be replaced with measuring the absorbance of DNA. Such methods are familiar to persons skilled in the art and typically involve determining the absorbance at 260nm.

Accordingly, in one example, the method for quantifying HIV DNA copy number further comprises:
(iii) quantifying the mass of DNA per volume (w/v) in the sample by measuring absorbance of DNA in the sample relative to a standard;
(iv) calculating the cell number per volume of DNA in the sample based on DNA absorbance; and
(v) normalising the HIV DNA copy number by calculating the HIV-R region DNA copy number per cell by dividing the value obtained in (ii) with the value obtained in (iv) to obtain the HIV R region DNA copy number (copies/cell) in the biological sample.

In a further example, the method of quantifying the mass of DNA per volume in the sample alternatively comprises the addition of a DNA intercalating dye and measuring the fluorescence emitted from the dye. In one example, the DNA intercalating dye is selected from SyBr Green I, Syto-9, Syto-10-14, Syto-16, Syto-21, Syto-24, Syto-29, YoYo-1, YoYo-3 and ToTo-1.

In another example, the HIV DNA copy number is expressed as the HIV-R region copy number per 1,000,000 cells (10⁶) i.e. copies/10⁶ cells. In another example, the HIV DNA copy number is expressed as a value for any fixed number of cells, including but not limited to 1,5 × 10⁶, 2 × 10⁶, 2.5 × 10⁶ or 3 × 10⁶ cells.

In one example, the volume is expressed as µl. In another example, the mass is the amount of DNA quantified by absorbance at 260nm. In another example, the mass is expressed as ng.

The methods described herein can be used to quantify the pVL of HIV-1 in the subject. Exemplified below are two methodologies which can be used to calculate pVL of HIV-1 in a subject (expressed as copies/10⁶ cells). These methods are to be considered as illustrative and not restrictive.

### Method 1 (based on Actin standard)

1. HIV-1 R region copy number is quantified by real-time PCR with an HIV-1 standard in a biological sample in which DNA is extracted (e.g. 42.5 copies/µl of extracted DNA) A
2. Actin copy number is also quantified by real-time PCR with an Actin standard using the same DNA extracted sample (e.g. 2,520,000 copies/µl of extracted DNA)
3. The cell number is then estimated from the Actin copy number divided by 200 (Actin has a copy number of 200 per cell) (e.g. estimated cell number is 12,600 cells/µl of extracted DNA) **B**
4. HIV-1 R region copy number per cell is calculated by dividing the value of A with the value of B **(A/B)** (e.g. estimated HIV-1 copy number per cell is 0.003373 copies/cell) **C**
5. HIV-1 R region copy number per 10⁶ cells is calculated by **C** × 1,000,000 (e.g. estimated HIV-1 copy number per 10⁶ cells is 3373 copies/10⁶ cells).

The HIV-1 standard is a plasma standard used to generate a standard curve (0, 4, 40, 4 ×10², 4 × 10³, 4 × 10⁴, 4 × 10⁵ and 4 × 10⁶ copy/µl). The Actin standards were used at 0, 20, 2 × 10², 2 × 10³, 2 × 10⁴, 2 × 10⁵ and 2 × 10⁶ copy/µl.

### Method 2 based on absorbance

An alternative method by which the level of HIV-1 can be measured is by using the absorbance at 260nm to determine the mass of DNA in the DNA extracted sample as follows:
1. HIV-1 R region copy number is quantified by real-time PCR with an HIV-1 standard in a biological sample in which DNA is extracted (e.g. 42.5 copies/µl of extracted DNA) A
2. Mass of DNA in the extracted DNA solution derived from patients sample is quantified by absorbance at 260nm (e.g. 111.8 ng/µl of extracted DNA)
3. Cell number is then estimated from absorbance of DNA. 1ng of DNA is estimated to be obtained after extraction of 150 cells (e.g. estimated cell number is 16673 cells/µl of extracted DNA) **B**
4. HIV-1 R region copy number per cell is calculated by dividing the value of A with the value of B **(A/B)** (e.g. estimated HIV-1 copy number per cell is 0.002535 copies/cell) **C**
5. HIV-1 R region copy number per 10⁶ cells is calculated by **C** × 1,000,000 (e.g. estimated HIV-1 copy number per 10⁶ cells is 2535 copies/10⁶ cells).

Thus, the HIV-1 DNA level determined by the above methods provides the clinician with the HIV-1 DNA level in a fixed number of cells.

### Method 3 based on use of DNA intercalating dye

A further method in which the level of HIV-1 can be measured is by use of a DNA intercalating dye.
1. HIV-1 R region copy number is quantified by real-time PCR with an HIV-1 standard in a biological sample in which DNA is extracted (e.g. 42.5 copies/µl of extracted DNA) **A**
2. Mass of DNA in the extracted DNA solution derived from patients sample is quantified by level of fluorescent emission of DNA intercalating dye (e.g. 111.8 ng/µl of extracted DNA)
3. Cell number is then estimated from fluorescent emission of the dye intercalated into DNA. 1ng of DNA is estimated to be obtained after extraction of 150 cells (e.g. estimated cell number is 16673 cells/µl of extracted DNA) **B**
4. HIV-1 R region copy number per cell is calculated by dividing the value of **A** with the value of **B (A/B)** (e.g. estimated HIV-1 copy number per cell is 0.002535 copies/cell)
5. HIV-1 R region copy number per 10⁶ cells is calculated by C × 1,000,000 (e.g. estimated HIV-1 copy number per 10⁶ cells is 2535 copies/10⁶ cells). **C**

Thus, the HIV-1 DNA level determined by the above methods provides the clinician with the HIV-1 DNA level in a fixed number of cells.

### Quantifying HIV RNA

The present disclosure also provides methods for quantifying HIV RNA, more particularly reverse transcribed HIV RNA (cDNA) in a biological sample. Accordingly, the present disclosure also provides a method of quantifying HIV RNA copy number in a biological sample obtained from a subject with HIV, or suspected of having an HIV infection, the method comprising:
(i) performing real-time PCR amplification of reverse transcribed HIV RNA (cDNA) in the sample using forward and reverse primers which hybridise to sequences within the R region of the long terminal repeats (LTRs) of HIV cDNA; and
(ii) quantifying the HIV-R region copy number per volume of RNA in the sample by reference to an HIV standard.

In one example, the method further comprises:
(iii) quantifying an endogenous housekeeping gene by quantitative PCR using a corresponding housekeeping standard to obtain the copy number for the endogenous gene, wherein the copy number is expressed as copies of housekeeping gene per volume of RNA present in the sample;
(iv) dividing the obtained copy number by the number of copies of the endogenous gene in a cell to derive the cell number per volume of RNA in the sample; and
(v) normalising the HIV RNA copy number by calculating the HIV-R region RNA copy number per cell by dividing the value obtained in (i) with the value obtained in (iii) to obtain the HIV R region RNA copy number (copies/cell) in the biological sample.

In one example, the method further comprises obtaining a biological sample from the subject and extracting RNA from the sample.

In one example, the housekeeping standard is amplified by real-time PCR.

The term "corresponding" as used herein is meant that the housekeeping standard is the same as the endogenous housekeeping gene.

In one example, the HIV-R region is quantified by:
(i) obtaining an aliquot of the sample containing extracted RNA;
(ii) contacting the aliquot with a labelled hydrolysis oligonucleotide which binds within the R region sequence of a long terminal repeat (LTR) of the reverse transcribed HIV RNA (cDNA);
(iii) further contacting the aliquot with forward and reverse primers which hybridise to sequences within the R region sequence of the reverse transcribed HIV RNA;
(iv) amplifying the R region sequence by quantitative PCR;
(v) extrapolating the signal obtained from the labelled oligonucleotide to a standard curve obtained by corresponding amplifications of serial dilutions of an HIV standard to derive the HIV-R region RNA copy number per volume of RNA in the aliquot.

In one example, the endogenous housekeeping gene is quantified by:
(i) obtaining an aliquot of an RNA sample prepared from the biological sample;
(ii) contacting the housekeeping gene in the aliquot with a labelled oligonucleotide which binds to a sequence within the housekeeping gene;
(iii) further contacting the aliquot with reverse transcriptase and forward and reverse primers which hybridise to sequences upstream and downstream respectively of the housekeeping gene sequence to which the oligonucleotide binds;
(iv) amplifying the reverse transcribed housekeeping gene sequence using quantitative PCR; and
(v) extrapolating the signal obtained from the labelled oligonucleotide to a standard curve obtained by corresponding amplifications of serial dilutions of the standard to derive the copy number per volume of DNA of the endogenous housekeeping gene in the aliquot.

In one example, the oligonucleotide is an hydrolysis oligonucleotide.

The method alternatively provides for quantifying HIV RNA copy number in a biological sample based on RNA absorbance. Accordingly, the present disclosure also provides a method for quantifying HIV RNA copy number in a biological sample from a subject with HIV, or suspected of having an HIV infection, the method comprising:
(i) detecting HIV-R region sequence;
(ii) quantifying the HIV-R region copy number per volume of reverse transcribed RNA (cDNA) in the sample by real-time PCR by reference to an HIV standard, wherein the copy number is expressed copies of HIV R region per volume of RNA in the sample;
(iii) quantifying the mass of RNA per volume in the sample by measuring absorbance of RNA in the sample;
(iv) calculating the cell number per volume of RNA in the sample based on RNA absorbance; and
(v) calculating the HIV-R region RNA copy number per cell by dividing the value obtained in (ii) with the value obtained in (iv) to obtain the HIV R region RNA copy number (copies/cell) in the biological sample.

In one example, detecting HIV-R region sequence is according to a method described herein or known in the art.

In one example, the mass is the amount of RNA quantified by absorbance at 260nm. In another example, the mass is expressed as ng.

In one example, the housekeeping gene is GAPDH.

In one example, the housekeeping RNA standard is a plasmid standard. In another example, the RNA standard is GAPDH. In another example, the RNA standard is serially diluted to provide 1, 20, 200, 2000, 20,000, 2 ×10⁵, and 2 ×10⁶ copies/µl.

In another example, the cell number is determined from the GAPDH copy number by dividing by 6.35 (assuming there are 6.35 copies of GAPDH RNA per cell). The person skilled in the art will appreciate that depending on the control RNA standard used, the value of 6.35 for GAPDH would need to be replaced with a different value corresponding to the number of copies of that gene in the cell.

In an alternative example, the method for quantifying HIV RNA copy number further comprises:
(iii) quantifying the mass of RNA per volume (w/v) in the extracted RNA solution derived from patients sample by measuring fluorescence emission of a RNA-intercalating dye.
(iv) calculating the cell number per volume of RNA in the sample based on the emission from the intercalating dye; and
(v) normalising the HIV RNA copy number by calculating the HIV-R region RNA copy number per cell by dividing the value obtained in in (ii) with the value obtained in (iv) to obtain the HIV-R region RNA copy number (copies/cell) in the biological sample.

In one example, the RNA copy number is expressed as the HIV R region copies/10⁶ cells.

### Detecting HIV transcription in latently infected reservoir cells

Methods described herein provide for detection and quantification of lentiviral transcription in latently infected cells. This is achieved by determining HIV-1 transcriptional activity normalised against HIV-1 DNA level.

The methods described above for calculating HIV-1 DNA can also be applied to the calculation of HIV-1 RNA. In this case, a GAPDH standard is used as shown in the examples. Real-time PCR is used to amplify RNA that has been reverse transcribed.

To obtain a value for the HIV transcriptional activity normalised against HIV DNA level, the HIV RNA level is divided by the HIV DNA level. This normalised HIV transcriptional activity (HIV-R region of RNA copy number divided by HIV-R region of DNA copy number) provides a measure of transcriptional activity occurring to identify active HIV-1 transcription. The advantage of this methodology is that we are able to access HIV transcriptional activity without sorting or separating of cells in the reservoir (namely CD4⁺ T cells and macrophages. This intracellular transcriptional assay is surprisingly useful to access efficacy of ART treatment, especially in patients whose plasma viral load is less than detection level (e.g. 20 copies/mL). The inventor was able to access and identify the HIV-1 transcriptional activity in the latently infected reservoir cells. In this way, the Applicant was able to separate subjects receiving optimal ART from subjects receiving sub-optimal ART.

Using this formula, a subject who is treated with ART can be monitored over time. If the treatment is effective, the subjects integrated HIV-1 DNA levels would decrease over the treatment period. Furthermore, if the subject is receiving optimal ART, the HIV-1 infection would not be expanding within the reservoir cells and accordingly the HIV-1 DNA integrated level would decrease.

Accordingly, the ratio of HIV-1 RNA copy numbers over HIV-1 DNA copy number provides the clinician with diagnostic data indicating current HIV-1 transcriptional activity normalised by HIV-1 DNA level at the time of sample collection. This gives a measure of how the ART treatment is affecting the latently infected cell population. Because the integrated HIV DNA level could be changed from patient to patient and it is essential to detect accurate HIV transcriptional activity normalized to the amount of HIV DNA.

### Assessing the effectiveness of anti-retroviral therapy

Without wishing to be bound by theory, the inventor has found that both HIV DNA copy number and normalised HIV RNA copy number provide a more sensitive method of separating subjects receiving ant-retroviral therapy (ART) versus those receiving sub-optimal/sub-effective ART. Simply measuring HIV RNA copy number did not discriminate between subjects, since the integrated HIV DNA level could be changed from patient to patient due to the initial HIV amount present in the patient when the patient is first infected or at a later infected stage. When the HIV RNA copy number was normalised relative the HIV DNA copy number, a statistically significant discrimination could be observed between subjects receiving optimal and sub-optimal ART. By performing the method over multiple time points, the normalised HIV RNA copy number can be monitored and compared to one or more previously determined values to assess whether the subject is receiving optimal ART. Therefore it is vital to detect accurate HIV transcriptional activity normalized to the amount of HIV DNA.

Accordingly the present disclosure also provides a method for assessing the effectiveness of anti-retroviral therapy (ART) administered to an HIV positive subject, the method comprising:
(i) quantifying the HIV-R region DNA copy number;
(ii) quantifying the HIV-R region RNA copy number;
(iii) determining a normalised HIV RNA copy number in a sample obtained from the subject by dividing the value obtained in step (i) with that obtained in step (ii); and
(iv) comparing the normalised HIV RNA copy number value to one or more previous normalised values obtained from the same subject;
wherein a decrease in the normalised HIV RNA copy number indicates that the subject is receiving optimal/effective ART.

In one example, the quantification of HIV-R region DNA or RNA is performed using a method described herein or known in the art.

In one example, the method further comprises obtaining a biological sample from the subject and preparing DNA and RNA from the sample.

In a further example, the HIV-R region RNA is quantified by:
(i) obtaining an aliquot of an RNA sample prepared from the biological sample;
(ii) contacting the aliquot with a labelled hydrolysis oligonucleotide which binds within the R region sequence of a long terminal repeat (LTR) of reverse transcribed RNA (cDNA);
(iii) further contacting the aliquot with reverse transcriptase and forward and reverse primers which hybridise to sequences within the reverse transcribed R region sequence upstream and downstream of the R region sequence to which the oligonucleotide binds;
(iv) amplifying the reverse transcribed R region (cDNA) sequence by quantitative real time DNA PCR;
(v) extrapolating the signal obtained from the labelled oligonucleotide to a standard curve obtained by corresponding amplifications of serial dilutions of an HIV standard to determine the HIV-R region RNA copy number per volume of RNA in the first aliquot.

In one example, the HIV standard used in the methods for quantifying HIV DNA and RNA is the same.

The method for quantifying RNA copy number has been described earlier. Preferably, the aliquots used for quantifying the endogenous housekeeping gene and quantifying HIV-R region RNA (cDNA) are derived from the same RNA prepared from the biological sample. Preferably, the quantification of HIV DNA and HIV RNA is carried out in the same biological sample from which DNA and RNA have been separately prepared/extracted.

In assessing whether the subject is receiving optimal ART, the clinical may look to compare the ratio of HIV RNA normalised by HIV DNA value with one or more previous values obtained from the same subject. If the ratio is decreasing over time, this would indicate to the clinician that the subject is receiving optimal ART. However, if the ratio increases then this would suggest to the clinician to either adjust the dosage of ART or the type of ART. This might also include substituting one of the components in the combination therapy if the subject is receiving combination therapy.

In a further example, the method comprises adjusting the dosage or type of ART administered to the subject.

In another example, the normalised HIV RNA copy number ratio is determined over at least two time points in the subject. In a further example, the ratio is determined over multiple time points over the life of the subject, including, but not limited to three, four, five, six, eight, ten, twelve, fifteen, twenty, twenty-five, thirty, thirty-five, forty etc. In another example, the period between the at least two time points is days, weeks or months. In another example, the period between the at least two time points is 1 week, 2 weeks, 1 month, 3 months, four months, six months, eight months, or twelve months .

In another example, a decrease in the ratio of greater than 50%, 45%. 40%, 35%, 30%, 25%, 20%, 15%, 10%, 8%, 5%, 2% indicates that the subject is receiving optimal ART.

### Anti-retroviral therapy and monitoring response to treatment

HIV RNA (viral load) and CD4 T lymphocyte (CD4) cell count are the two surrogate markers of antiretroviral treatment (ART) responses and HIV disease progression that have been used for decades to manage and monitor HIV infection.

Viral load is a marker of response to ART. A patient's pre-ART viral load level and the magnitude of viral load decline after initiation of ART provide prognostic information about the probability of disease progression. The key goal of ART is to achieve and maintain durable viral suppression. Thus, the most important use of the viral load is to monitor the effectiveness of therapy after initiation of ART.

Measurement of CD4 count is particularly useful before initiation of ART. The CD4 cell count provides information on the overall immune function of an HIV-infected patient. The measurement is critical in establishing thresholds for the initiation and discontinuation of opportunistic infection (OI) prophylaxis and in assessing the urgency to initiate ART.

### CD4 Count Monitoring

The CD4 count is the most important laboratory indicator of immune function in HIV-infected patients. It is also the strongest predictor of subsequent disease progression and survival according to findings from clinical trials and cohort studies. CD4 counts are highly variable; a significant change (2 standard deviations) between 2 tests is approximately a 30% change in the absolute count, or an increase or decrease in CD4 percentage by 3 percentage points.

HIV infection is characterized by high rates of viral turnover throughout the disease process, eventually leading to CD4 depletion and disease progression. (Wei X, Ghosh S K, Taylor M E, et al. (1995) Nature 343, 117-122 and Ho D D, Naumann A U, Perelson A S, et al. (1995) Nature 373, 123-126). The aim of antiretroviral therapy is to achieve substantial and prolonged suppression of viral replication. Achieving sustained viral control is likely to involve the use of sequential therapies, generally each therapy comprising combinations of three or more antiretroviral drugs. The primary rationale of combination therapy relates to synergistic or additive activity to achieve greater inhibition of viral replication. The tolerability of drug regimens will remain critical, however, as therapy will need to be maintained over many years.

In an untreated patient, some 10¹⁰ new viral particles are produced per day. Coupled with the failure of HIV reverse transcriptase (RT) to correct transcription errors by exonucleolytic proofreading, this high level of viral turnover results in 10⁴ to 10⁵ mutations per day at each position in the HIV genome. The result is the rapid establishment of extensive genotypic variation.

Early development of antiretroviral therapy has focused on inhibitors of reverse transcriptase. Both nucleoside and non-nucleoside inhibitors of this enzyme have shown significant antiviral activity (DeClerq, E. (1992) AIDS Res. Hum. Retrovir. 8:119-134). However, the clinical benefit of these drugs had been limited due to drug resistance, limited potency, and host cellular factors (Richman, D. D. (1993) Ann. Rev. Pharm. Tox. 32:149-164).

More recent therapies have focussed on the use of protease inhibitors. These include agents such as Saquinavir, developed by Hoffmann-La Roche, Ritonavir, developed by Abbott Laboratories, Indinavir, developed by Merck & Co., Nelfinavir, developed by Agouron Pharmaceuticals and Amprenavir developed by Vertex Laboratories. Additional protease inhibitors include atazanavir, darunavir, fosamprenavir, and tipranavir.

Other classes of drug that may be used for treatment include fusion inhibitors such as Fuzeon, CCR5 co-receptor antagonist such as selzentry and HIV integrase strand transfer inhibitors such as isentress, tivicay and vitekta.

Combination antiviral therapy with protease and reverse transcriptase inhibitors has demonstrated the potential therapeutic efficacy of antiviral therapy for treatment for AIDS. Such combinations include atripla, complera, evotaz, prezcobix and stribild.

It will be appreciated by persons skilled in the art that numerous variations and/or modifications may be made to the above-described embodiments, without departing from the broad general scope of the present disclosure. The present embodiments are, therefore, to be considered in all respects as illustrative and not restrictive. The invention is as defined in the appended claims. The present disclosure includes the following non-limiting examples.

### Exam ples

### Materials and Methods

### Design of primer and probe sequences

The genomic structure of HIV-1 is shown in Figure 1. The genomic structure of HIV-2 is shown in Figure 2. Primer sets were designed to target identical "R" region sequences located within the 5'LTR and 3'LTR of the HIV-1 or HIV-2 genome. Primer sets were designed based on the following criteria (a) located in a conserved region in all HIV-1 and HIV-2 subtypes for reliable detection, (b) primer length of between 18-25 bases long, and (c) no primer dimer formation but maintaining sensitive detection. The forward primer was designed to provide good alignment at the 3' end of the primer for all HIV subtypes and the reverse primer was designed to provide good alignment at the 5' end of the primer for all HIV subtypes. The forward and reverse primers are show in Table 1 below.

**Table 1 Sequences of forward and reverse primers for PCR**

| **Forward primer 5' to** 3' **orientation** | **Reverse primer 5' to 3' orientation** | **SEQ IDs** |
|---|---|---|
| HIV-1 | HIV-1 | 7 and 9 |
| GAGCCTGGGAGCTCTCTG | ACTCAAGGCAAGCTTTATTGAGGC | |
| HIV-2 | HIV-2 | 14 and 16 |
| GAGCCCTGAGAGGTTCTC | GGTCTTTAAGCAAGCAAGCGTGG | |
| HIV-2 For 2 | HIV Rev 2 | 33 and 34 |
| GAGCCCTGRGAGGTTCTC | AGGGAACACCCAGGCTCT | |
| R refers to a G/A mix wherein the proportion of G is 90% and the proportion of A is 10%. | | |
| HIV-2 For 3 | HIV-2 Rev 3 | 35 and 36 |
| TTGAGCCCTGGGAGGTTCT | GGAACACCCAGGCTCTAC | |
| GAPDH | GAPDH | 18 and19 |
| GGCAACAATATCCACTTTACCAG | TCGACAGTCAGCCGCATCTT | |
| Actin | Actin | 21 and 22 |
| TCACCCACACTGTGCCCATCTACGA | CAGCGGAACCGCTCATTGCCAATGG | |

Taqman^{®} probes designed to bind within a conservative region in all HIV subtypes to ensure efficient detection. Lock Nucleic Acid (LNA) modification was employed to allow an increase in the annealing temperature and the use of short TaqMan probes.

The sequence of the HIV-1oligonucleotide probe was as follows:
5' FAM-AGG_{LNA}GA_{LNA}AC_{LNA}CCAC_{LNA}TG_{LNA}CTTA-BQH-1 3' (SEQ ID NO:6) wherein FAM is the fluorescent reporter and BHQ1 is the quencher molecule and N_{LNA} represents a locked nucleic acid analogue where N is the indicated A, T, G or C nucleobase.

The sequence of the HIV-2 oligonucleotide probe No. 1 was as follows:
5' FAM- GCCTGGGTGTTCCCTGCTAGACTCT -BQH-1 3' wherein FAM is the fluorescent reporter and BHQ1 is the quencher molecule.

The sequence of the HIV-2 oligonucleotide probe No. 2 was as follows:
5' FAM- CT_{LNA}GC_{LNA}TA_{LNA}GT_{LNA}G_{LNA}CTGG_{LNA}A -BQH-1 3' (SEQ ID NO:40) wherein FAM is the fluorescent reporter and BHQ1 is the quencher molecule and N_{LNA} represents a locked nucleic acid analogue where N is the indicated A, T, G or C nucleobase.

The sequence of the HIV-2 oligonucleotide probe No.3 was as follows:
5' FAM TC_{LNA}CA_{LNA}GC_{LNA}AC_{LNA}TA_{LNA}GC_{LNA}AG -BQH-1 3' (SEQ ID NO:44) wherein N_{LNA} represents a locked nucleic acid analogue where N is the indicated A, T, G or C nucleobase.

Alignment of the probes and PCR primers used herein in the HIV-R region sequence for HIV-1 and HIV-2 are shown in Figure 3A-D. Figure 3A represents the alignment on HIV-1 and Figures 3B-D represent the alignment on HIV-2.

The alignment of the HIV-1 oligonucleotide probe for HIV-1 subtypes is shown in Figure 4.

The alignment of the oligonucleotide probe for HIV-2 subtypes for HIV-2 probes is shown in Figure 5A-C.

The sequence of the GAPDH probe was 5' FAM-AAGGTCGGAGTCAACGGATTTGGTCGT- BQH-1 3' (SEQ ID NO:17) wherein FAM corresponds to the fluorescent reporter and BHQ1 is the quencher molecule.

The sequence of the β-actin probe was 5' FAM-ATGCCCTCCCCCATGCCATCCTGCG BHQ-1 3' (SEQ ID NO:20).

### Labelling of primers and probes

All primers were synthesised by standard commercial oligo-DNA synthesis service. Biotin-labelled primers were used for End-Point PCR detection. The biotin modification was made at 5'-end of oligo-DNA primer.

### Cell lines and plasmid standards

HIV-1 plasmid standard used herein was obtained from genomic HIV-1 plasmid of pNL4-3 (NIH Cat No. 114). TA-cloned HIV-2 plasmid standard was generated by standard procedure. At first HUT78 cells were infected with HIV-2 CBL-20 virus [NIH Cat No 600). Genomic DNA was isolated followed by PCR amplification. T/A cloning HIV-2 was obtained from this amplicon to generate a plasmid which contained HIV-2 R-region.. The HIV-2 Plasmid copy number was determined using spectrum at 260nm absorbance and molecular weight of each plasmid.

HUT78 cell (CD4+ T cell line, Cat No. 89) and OM10.1 (HIV-1 latent cell line, Cat No. 1319) were also obtained from NIH. OM10.1 cell line contains a single copy of HIV-1 genomic DNA in one single OM10.1 cell.

### HIV subject data

Plasma viral load data for subjects was obtained from the St Vincent's Hospital diagnostic laboratory. Data was obtained regarding a subject's HIV status using the Roche COBAS^{®} TaqMan HIV-1 assay, according to manufactures' instructions. Subjects were adult subjects over the age of 18 and informed consent was obtained to use their blood samples.

### Isolation and preparation of peripheral blood mononuclear cells (PBMCs)

Whole blood was obtained from subjects using standard protocols for blood collection. Fresh blood not older than 4 days was obtained from the HIV diagnostics lab at St Vincent's Hospital.

Peripheral blood mononuclear cells (PBMCs) were isolated from whole blood using the conventional Ficoll gradient centrifugation method. Briefly, blood was collected in 9mL Acid Citrate Dextrose anticoagulant (ACD) tubes and 9mL of phosphate buffered saline (PBS) was added. After mixing by inversion, 15mL of Ficoll-plaque media was added and centrifuged at 400g for 40 mins at 18° to 20° C. The upper layer containing plasma and platelets was discarded and the mononuclear layer of cells transferred to a clean tube. 30mL of PBS was added, mixed and centrifuged at 400g for 15 mins at 18° to 20° C. The supernatant was removed and the mononuclear cells washed in 10mL PBS and centrifuged at 400g for 10 mins at 18° to 20° C. The supernatant was removed and mononuclear cells retained for future analysis.

### Isolation and preparation of whole blood

For whole blood experiments, 4 mL of Red blood Cell lysis buffer (Roche) was added to a 15mL Falcon tube. The whole blood collection tube was inverted 10 times and then 2 mL of whole blood added to the 15mL Falcon tube containing 4mL of the Lysis buffer. The tubes were incubated on a rotating platform with slow rotation mode at room temp (RT) for 10min. The tube was then centrifuged at 500xg for 5 min with a table-top centrifuge at RT. After centrifugation, supernatant was decanted into a waste bottle. Cells were resuspended in 1800µl of PBS. Two aliquots of 900 µl of cell suspension were made into two O-ring tubes.

The tubes were then centrifuged at 7,800xg (9000rpm) for 3 min with a microfuge at RT. Supernatant was removed without disturbing the white cell pellets using P1000 pipette.

Two cell-pelleted tubes were prepared from one patient. One tube is for DNA extraction and the other tube for RNA extraction.

The concentration of extracted DNA in the sample was measured by absorbance at 260nm (A₂₆₀) utilising an average extinction coefficient for double-stranded (ds) DNA (1A₂₆₀=50 µg/ml) to determine the nucleic acid concentration from the absorbance of the nucleic acid preparation.

### Preparation of PBMCs

PBMCs were obtained by Ficol-Paque separation method from whole blood. The Patient's Blood was collected in 9mL ACD (Acid Citrate Dextrose, anticoagulant) tube. 9 mL of the blood was transferred into 50mL Falcon tube and 9mL of PBS added. The blood and buffer were mixed by inverting the tube several times. 15mL of Ficoll-Paque media was added at the bottom of the 50mL of Falcon tube with very slowly without interfering with the interface between Ficoll-Paque media and Blood phase. The tube was centrifuged at 400g for 40 min at 18°C to 20°C (brake of the centrifuge was turned off). The upper layer was drawn off the plasma and platelets using a sterile pipette, leaving the mononuclear cell layer undisturbed at the interface. The layer of mononuclear cells was then transferred to a 50ML of Falcon tube. 30mL of PBS was added to the cells and mixed by inverting the tube several times. The tube was centrifuged at 400g for 15 min at 18°C to 20°C (with brake-on). The supernatant was removed. The mononuclear cells were re-suspended in 10 ml of PBS. Centrifuge at 400 × g for 10 min at 18°C to 20°C. The supernatant was then removed.

### Purification of White blood cell sub-types

For purification of RO+R5+, RO+R5- and RO-R5- cell populations, the PBMC cells were sorted by a FACS Aria cell sorter (BD Biosciences) based on surface marker on PBMCs.

PBMCs (about 2 × 10⁶ cells) were incubated with mAb's against CD4 (PerCP; Becton Dickinson), and CD3 (APC; Becton Dickinson), CCR5 (clone 2D7, FITC conjugated; PharMingen), CD45 RO (PE; PharMingen). Cells were incubated with marker for 30 min at room temperature. The tubes were then centrifuged at 400g for 5 min and the cells resuspended in 1mL of PBS with 10% FCS. This was repeated two more times and the cells resuspended in 1mL of PBS with 10% FCS. Cells were sorted by "Aria cell sorter" to get following three T-cell subset populations
CD3+ CD4+ CD45RO+ ccr5+
CD3+ CD4+ CD45RO+ ccr5-
CD3+ CD4+ CD45RO- ccr5-.

Separation of PBMCs bearing CD14 was performed using magnetically labelled particles (Dynabeads^{®}) according to manufacturer's instructions.

### DNA extraction

Genomic DNA was extracted using the QIAamp DNA mini kit (Qiagen) according to manufactures' instructions.

Briefly, to the cell pellets was added 200µl of AL buffer (provided in the kit) and the tubes vortexed until the pellet dissolved. A Proteinase-K master mix was prepared.

| | |
|---|---|
| 200µl of PBS | x number of tubes |
| 20µl of Proteinase K | x number of tubes |

220µl of Proteinase-K master mix was added and the tubes vortexed. DNA was digested at 56°C for 10 min followed by brief voltex every 2-3 min. Tubes were then centrifuged briefly and add 200 µl of EtOH added, followed by a voltex for 15 sec. Tubes were then centrifuged briefly and all lysed solution added to the QIAamp DNA Minicolumn and centrifuge at 6,000xg for I min at RT. The upper part of QlAamp column was transferred into the new the liquid collection tube (provided in the kit). The used lower part of the collection tube was discarded into waste. 500µl of AW1 buffer (provided in the kit) was added and centrifuges at 6,000xg for I min at RT. The upper part of QlAamp column was transferred into the new liquid collection tube (provided in the kit). The used lower part of the collection tube was discarded into waste. 500µl of AW2 buffer was added and centrifuges at 13,000xg for 3 min at room temperature.

The liquid from the collection tubes was emptied and an additional centrifuge carried out at 13,000xg for 1 min at room temperature. The upper column was removed and placed into 1.5mL eppendorf tubes. 60µ AE buffer (provided in the kit) was added and kept for 1 min at RT, followed by a centrifuge at 6,000xg for 1 min at RT. The used upper part of the collection tube was discarded and the lower part of the centrifuge tubs stored at 4°C.

### RNA extraction

Total RNA was extracted from blood samples using the ReliaPrep^{™} RNA cell system (Promega) according to manufactures' instructions.

Briefly, 250ul of BI+TG buffer (provided in the kit) was added to each cell pellet. Tubes were vortexed until pellets are dissolved. 85ul of Isopropanol was added and the tubes vortexed. The lysed solution was transferred to the ReliaPrep Minicolumn (provided in the kit) and centrifuged at 14,000xg for I min at room temperature. Liquid from the collection tubes was emptied into waste.

DNA-1 master mix was prepared in the new tubes to get necessary volume for all tubes.

| | | |
|---|---|---|
| 24µl of Yellow Core Buff | x number of tubes | |
| 3µl 0.09M MnCI2 | x number of tubes | |
| 3µl of DNase I enzyme. | x number of tubes | Mix well this tube |

30ul of DNA-1 master mix was added on the filter of the ReliaPrep Minicolumn and incubated for 15 min at RT. 200ul of the Column Wash Solution (provided in the kit) was added and centrifuged at 14,000xg for I min at room temperature. 500µl of RNA Wash Solution (with ethanol added) and centrifuged at 14,000xg for I min at room temperature. The ReliaPrep^{™} Minicolumn was placed into a new Collection Tube and 300µl of RNA Wash Solution added (provided in the kit). The tube was centrifuged at maximal speed for 2 min at RT and then removed into 1.5mL eppendorf tubes. Add 60µl Nuclease-Free Water was added and the tubes kept for 1 min at RT, followed by a centrifuge at 13,000xg for 1 min at RT. The used upper part of the collection tube was discarded and the lower part of centrifuge tubes stored at 4°C.

All DNA and RNA samples were analysed by spectrophotometer to confirm quality using Qubit Fluorometric Quantitation (ThermoFisher).

### DNA amplification real-time assay

The real-time PCR method used in the present disclosure using a set of PCR primers and TaqMan^{™} probe is shown schematically in Figure 6A and B. Two sets of real-time PCR were used for DNA analysis using the SensiFAST Probe No-ROX One-step kit (BIO76005). Each reaction mixture in a 40 µl final volume contained: 2× PCR buffer, 20 µM forward primer, 20 µM reverse primer, 6 µl of DNA, 5 µM Taqman probe and PCR-grade water. The cycling and melting conditions were as follows: 94°C for 30 seconds followed by 50 cycles of: 7 seconds at 95°C and 30 seconds at 60°C using the LightCycle 480 (Roche). The HIV-1 DNA standard was serially diluted to provide 0, 40, 400, 4,000, 40,000, 4 × 10⁵ and 4 × 10⁶ copies/µl.

For β-actin analysis, the reaction mixture in a 40 µl final volume contained: 2× PCR buffer, 20 µM forward primer, 20 µM reverse primer, 6 µl of DNA, 5 µM Taqman probe and PCR-grade water. The cycling and melting conditions were as follows: 94°C for 30 seconds followed by 50 cycles of: 7 seconds at 95°C and 30 seconds at 60°C using the LightCycle 480 (Roche).]. The beta actin standard was serially diluted to provide 0, 20, 200, 2,000, 20,000, 2 × 10⁵ and 2 × 10⁶ copies/µl. The beta actin standard was a plasmid containing a single copy of the beta actin gene. The beta actin plasmid standard was made by TA cloning using PCR amplified beta actin DNA and standard cloning methods. The beta-actin primer and probe sequences are as described above.

The HIV-1 cloned plasmid was obtained from the NIH, one single plasmid contains one single copy of HIV-1 genome.

### RNA amplification real-time assay

Two sets of real-time PCR were used for RNA analysis using the SensiFAST Probe No-ROX One-step kit (BIO76005). For RNA analysis, the reaction mixture in a 40 µl final volume contained: 2× PCR buffer, 20 µM forward primer, 20 µM reverse primer, 6 µl of RNA, 5 µM Taqman probe, 0.45 µl reverse transcriptase, 0.8 µl RNase Inhibitor and PCR-grade water. The cycling and melting conditions were as follows: 45°C for 20 mins, 94oC for 2 mins followed by 50 cycles of: 7 seconds at 95°C and 30 seconds at 60°C using the LightCycle 480 (Roche). Each PCR amplification was performed in triplicate unless indicated otherwise.

For glyceraldehyde 3-phophate dehydrogenase (GAPDH) analysis, the reaction mixture in a 40 µl final volume contained: 2× PCR buffer, 20 µM forward primer, 20 µM reverse primer, 6 µl of RNA, 5 µM Taqman probe, 0.45 µl reverse transcriptase, 0.8 µl RNase Inhibitor and PCR-grade water. The cycling and melting conditions were as follows: 45°C for 20 mins, 94°C for 2 mins followed by 50 cycles of: 7 seconds at 95oC and 30 seconds at 60°C using the LightCycle 480 (Roche).

The GAPDH standard was serially diluted to provide 0, 20, 200, 2,000, 20,000, 2 × 10⁵ and 2 × 10⁶ copies/µl. The GAPDH standard was a plasmid containing a single copy of the beta actin gene. The GAPDH plasmid control was made by TA cloning using PCR amplified GAPDH DNA by standard cloning methods.

The GAPDH primer and probe sequences are as described above.

### End-point PCR assay

The inventor has developed an end-point PCR assay referred to herein as the Streptavidin (SA)-plate end-point assay. This assay is shown schematically in Figure 6B and is performed using a biotin-labelled primer. Following PCR, the PCR product is hybridised with a digoxigenin (Dig) labelled probe. The newly hybridised products are captured on the surface of a streptavidin coated 96-well plate. After the unbound Dig probe is washed out, a peroxidase (POD)-labelled antibody against Dig is added followed by a chemiluminescent reaction. The release light is then detected by a luminescent plate reader.

All PCR reactions are conducted in the same manner as in TaqMan based Real-Time PCR method (see "DNA amplification real-time assay" and "RNA amplification real-time assay" above), except the following two conditions: i) a TaqMan probe is not used, which is replaced by the Water in the reaction, and ii) one of the set of primers is biotin-labelled at 5'- end of oligo DNA. After PCR is completed, 10µl of PCR amplicon is transferred to a PCR tube containing 40 µl of a hybridization buffer of 20pmole of a Dig-labelled probe and 3.85XSSC buffer (e.g. 20X SSC contains 3.0M NaCl and 0.3M sodium citrate pH7.0). In order to hybridize PCR amplicon and Dig-labelled probe, the temperature of the solution of this PCR tube is elevated at 95°C for 2min, then 40°C for 2min, followed by 4°C. Total volume of 50µl hybridized products is transferred to streptavidin coated 96-well-plate, which contained 50µl of a dilution buffer of 10mM Tris-HCl pH7.6, 150mM NaCl, 1mM EDTA, and 0.0002% Tween20. The 96-well plate is incubated at 37°C for 30 min. The hybridized products of the biotin-labelled PCR amplicon and the Dig-probe are bound to the streptavidin plate during this incubation. Unbound products was washed out from the plate during 3X wash with Wash buffer (10mM Tris-HCl pH7.5, 0.15M NaCl, 1mM EDTA, 0.01% Tween20). Following which 100µl of Peroxidase conjugated anti-Dig-Antibody (1 in 5000 dilution) using a dilution buffer of 5% BSA 10mM Tris-HCl pH7.5, 0.15M NaCl is added. The 96-well plate is then incubated at 37°C for 30 min, followed by 6X wash using Wash buffer. 100µl of Chemiluminescent Peroxidase Substrate (CPS260, Sigma) is then added. The palate is then incubated at Room Temperature for 5 min. The light generated from the substrate is detected with a luminescent plate reader to get Relative Light Unit (RLU). A gain (adjustment value) of 1200 is used for the luminescent plate reader.

### Example 1 HIV-1 RNA copy number (pVL) normalised by HIV-1 DNA copy number

Samples from forty seven (47) HIV-1 subjects undergoing ART were obtained and DNA and RNA extracted from whole blood cell lysates as detailed above. At the time of collection, the plasma viral load (pVL) in all patients was suppressed and was >20 copies/mL (Figure 7).

HIV-1 patients were assigned as either receiving "optimal ART" or "sub-optimal ART" based on the plasma viral load over the past 6 months. Patients with a pVL of <20 copies/mL for the past 6 months were considered to have "optimal ART", whilst patients having occasionally elevated pVL (i.e., >20 to <200 copies/mL; referred to a blip (BL)) during the last 6 month period were designated as receiving "sub-optimal ART".

Quantitation of HIV-1 RNA and DNA based on amplification of the HIV-1 R region sequence was carried out by real-time PCR analysis as described according to the above protocols using a beta actin standard for DNA and GAPDH for RNA. Forty seven samples obtained from the SVH diagnostics laboratory were analysed.

The RNA plasma viral loads (pVLs) of these 47 samples were totally suppressed at the time of collection (< 20 copies/mL). As can be seen in Figure 7, there was no difference in both the optimal and sub-optimal ART subjects using the standard pVL assay.

When the HIV-1 RNA copy number was normalised to HIV-1 DNA copy number as shown in Figure 8, the assay could discriminate subjects receiving optimal ART from those receiving sub-optimal ART since a statistically significant difference (p=0.0016) was found in the intracellular viral activities between the "optimal ART" (n=29) and "sub-optimal ART" (n=18) subjects (Figure 8A). Subjects who had previously experienced a viral load blip (BL), namely at least one episode of transient high HIV-1 DNA level, as well as four subjects with immunological failure (IF) are identified in the sub-optimal ART category as having a significantly higher mean ratio of normalised HIV-1 RNA (Figure 8B). Subjects whose pVL had been suppressed for greater than 6 months had a mean lower ratio of normalised HIV-1 RNA.

Accordingly, the inventor's assay was able to discriminate between subjects receiving optimal or sub-optimal ART based on normalisation despite the pVL RNA assay showing no difference between these two groups of subjects.

As shown in Figure 8C, a further category, designated "Improved Optimal ART" was seen in subjects having a HIV-1 DNA copy number less than 800 HIV-1 DNA per 10⁶ cells according to the inventors assay which was statically significant (p=0.012) compared to the rest of the Optimal ART group.

The data demonstrates that HIV activity in the latently infected reservoir can be detected using primers located in the 5' and 3' LTR R region and that determining intracellular HIV-1 copy number as shown herein provides a more sensitive indicator of treatment response than standard pVL. Furthermore, as the level of RNA transcription is normalised to the level of integrated DNA, the increase in intracellular HIV load indicates that latent viral reservoir is transcriptionally active.

The results also suggest that both a low ratio of normalised HIV-1 RNA (i.e. low transcriptional activity) as well as low HIV-1 DNA in a subject is indicative of improved-optimal ART. Accordingly, the clinician might look to both of these measures, namely ratio of normalised HIV-1 RNA (less than 0.05) and a HIV-1 DNA copy number of less than 800 HIV-1 DNA per 10⁶ cells when determining whether a subject is receiving appropriate (i.e. optimal) ART.

### Example 3 Evaluation of HIV-1 R region DNA detection

Two subjects (referred to in Table 3 as A and B) who were known to be infected with HIV-1 were evaluated in this study. HIV-1 infected Peripheral blood mononuclear cells (PBMCs) from the subjects were sorted by flow cytometry based on expression of the markers CD3+, CD4+, CD45RO+, CD45RO- and CCR5+, which have previously been demonstrated to contain an enriched population of infected cells.

Briefly, PBMCs were sorted using a FACS ARIA cell sorter (BD Biosciences) based on cell surface markers according to manufacturers' instructions. Briefly, 2×10⁶ cells were incubated with monoclonal antibodies against CD4 (PerCP, Becton Dickinson), CD3 (APC, Becton Dickinson), CCR5 (clone 2D7, FITC conjugated; PharMingen) and CD45 RO (PE; PharMingen). CD45 RO+ cells generally distinguish memory T cells, while CD45 RO- cells generally distinguish naiive T cells. CD3 is a pan T cell marker which is used to separate T cells from non- T cells. CD4, is generally found on T helper cells, monocytes, macrophages and dendritic cells. CCR5 is a chemokine receptor found on white blood cells and which is often used by HIV to infect white blood cells.

Cells were incubated for 30 mins at room temperature and centrifuged at 400g for 5 mins. Cells were washed 3 times in 1mL PBS/10%FCS. Washed cells were sorted to isolate the following T cell subsets:
- CD3+ CD4+ CD45 RO+ CCR5+
- CD3+ CD4+ CD45 RO+ CCR5-
- CD3+ CD4+ CD45 RO- CCR5-

The samples were analysed using primers sets against three different intracellular locations within the HIV-1 DNA:
(1) the 5' and 3' LTR R region,
(2) the 3'LTR region
(3) the gag region.

The 5' and 3' LTR R region primers are as described earlier herein.

The 3'LTR amplification forward and reverse primers were respectively 5'-CCAAAGAAGACAAGATATCCTTGA-3' (SEQ ID NO:23) and 5'- TTGAGGCTTAAGCAGTGG - 3' (SEQ ID NO:24). The sequence of the oligonucleotide probe was 5'- FAM-TTAGACCAGATCTGAGCCTGGGAGCTCTC-BHQ1 -3' (SEQ ID NO:25) where FAM is fluorescent labelled dye and BQ1 is quencher of this fluorescent dye. This primer set amplifies a region spanning the U3 and R regions of the 3' LTR.

The gag forward and reverse primers were respectively 5' AGTGGGGGGACATCAAGCAGCCATGCAAAT 3' (SEQ ID NO:26) and 5' TACTAGTAGTTCCTGCTATGTCACTTCC 3' (SEQ ID NO:27). The sequence of the oligonucleotide probe was 5'- FAM- ATC_{LNA}A_{LNA}ATG_{LNA}AGGAAG_{LNA}CTG_{LNA}C-BHQ1 -3' (SEQ ID NO:28) wherein FAM corresponds to the fluorescent reporter and BHQ1 is the quencher molecule. Locked nucleic acids are indicated by N_{LNA} where N is the indicated A, T, G or C nucleobase.

The results are shown in Table 3 below. The R region primers detected intracellular HIV-1 DNA in unsorted samples from both subjects. Following cell sorting, detection of HIV-1 DNA using the 3'LTR and gag primers was variable in subject A, whilst both primer sets failed to detect HIV-1 DNA in subject B. In contrast, the R region primers detected HIV-1 DNA in all samples of subject A and at low levels in the CD3+ CD4+ CD45RO- CCR5- in subject B.

These results indicate that detection of HIV-1 DNA is more sensitive using primers directed to the R region compared with detection based on non-R region amplification.

**Table 3: Evaluation of HIV-1 R region DNA detection**

| Subject **ID** | Sample | Intracellular HIV-1 DNA copy **number/10⁶** GAPDH copies | | |
|---|---|---|---|---|
| | | 3'LTR primers | gag primers | R region primers |
| A | Unsorted PBMCs | 91.25 | 0 | 95.62 |
| | CD3+ CD4+ CD45 RO+ CCR5+ | 327.26 | 115.84 | 822.49 |
| | CD3+ CD4+ CD45 RO+ CCR5- | 278.35 | 275.46 | 298.58 |
| | CD3+ CD4+ CD45 RO- CCR5- | 34.95 | 0 | 180 |
| | | | | |
| B | Unsorted PBMCs | 0 | 0 | 9.53 |
| | CD3+ CD4+ CD45 RO+ CCR5+ | 0 | 0 | 0 |
| | CD3+ CD4+ CD45 RO+ CCR5- | 0 | 0 | 0 |
| | CD3+ CD4+ CD45 RO- CCR5- | 0 | 0 | 17.47 |

### Example 4: Evaluation of HIV-1 R region RNA detection sensitivity

To evaluate HIV-1 RNA in patient samples, eight (8) subjects known to be HIV-1 positive were selected and analysed using primers sets against three different intracellular locations: (1) the 5' and 3' LTR R region, (2) the 3'LTR region and (3) the gag region, similar to the HIV-1 DNA analysis in Example 3.

Primers to the 5' and 3' LTR R region, 3'LTR region and the gag region were used as previously described.

In contrast to Example 3 above, analysis was conducted on cells isolated from whole blood as described earlier. RNA extraction and PCR amplification was performed as previously described.

As shown in Table 4 below, primers located in R region at both the 5' and 3' LTR were able to detect intracellular HIV transcript in all eight subjects, whilst primers located in the 3'LTR and gag regions only detected HIV load in four and three subjects respectively.

This data demonstrates that primers located in R region at both the 5' and 3' LTR were more sensitive to detection of intracellular DNA copy number when compared to primers located in the 3'LTR and gag regions. Despite those subjects having a VL less than the detection level, consistent detection of intracellular HIV-1 transcriptional activity based of R region was detected.

**Table 4: Evaluation of HIV-1 R region RNA detection**

| **Subject ID** | **pVL (copy/ml)** | **Intracellular copy number /10⁷ GAPDH RNA copies** | | |
|---|---|---|---|---|
| | | **3' LTR** | **gag** | **R** |
| 1 | <20 | 32.8 | 0.0 | 91.8 |
| 2 | <20 | 94.2 | 22.8 | 42.6 |
| 3 | <20 | 0 | 0 | 7.8 |
| 4 | <20 | 5.1 | 0.3 | 6.1 |
| 5 | <20 | 0 | 0 | 12.6 |
| 6 | <20 | 10.7 | 7.5 | 23.7 |
| 7 | <20 | 0.0 | 0.0 | 12.7 |
| 8 | <20 | 0 | 0 | 47.3 |

### Example 5: Intracellular HIV load is indicative of persistent HIV transcriptional activity and contributes to plasma viral load rebound

To investigate whether the low but persistent levels of intracellular HIV load were indicative of HIV transcriptional activity, subjects were sampled during anti-retroviral therapy (ART) and during a structured treatment interruption (STI) period (i.e., no ART treatment).

Subjects were analysed for plasma viral load and viral RNA at 12 week intervals during ART. Following cessation of therapy, subjects were sampled at intervals for up to 16 weeks. Intracellular transcriptional activity was detected using R region assays as previously described.

Four Treatment naive patients were treated with ART for one year. The level of plasma Viral Load (pVL, line with circles) in four patents was under the detection limit during 24 week long treatment (Figure 9A, B, and D) and during 12 week long treatment (Figure 9C) The pVL level was kept under the detection level until week 52 (one year) of ART treatment. Despite undetectable pVL in the four patents, low level of intracellular HIV-1 transcriptional activity was detected (grey column). After stopping therapy there was rapid elevation of the VL in four patients and elevated intracellular HIV-1 transcriptional activity was accompanied with increased VL.

ART treatment period is indicated in the grey horizontal bar and Stricture Treatment Interruption (STI: stopping ART) period is indicated in the white horizontal bar (Figure 9).

Subjects in Figure 9 went into the second phase of the ART treatment (Figure 10). During this second phase ART period, VL was suppressed under detection level, however, consistent detection of intracellular HIV-1 transcriptional activity even with VL under detection level (grey column). After stopping the second phase of ART treatment, a quick rebound VL was observed in both subjects (Figure 10A and B).

To investigate whether the increase in transcriptional activity was derived from cells in the latently infected reservoir, intracellular RNA at baseline (pre-ART) and at week 2 after stopping ART (grey circles on the horizontal axis), and rebounded HIV-1 plasma RNA at week 16 post-ART (indicated by upper grey circle) were collected and analysed by Sanger sequencing.

Data was analysed using a neighbour joining clustering method on a web-based HIV database (http://www.hiv.lanl.gov/content/sequence/HIV/HIVTools.html). Samples isolated from the same subjects clustered together (grey rectangle box) suggesting that the latent viral reservoir has persistent HIV transcriptional activity that contributes to the plasma viral load rebound (Figure 11).

### Example 6 Evaluation of HIV-2 copy number in plasma

Two plasma samples containing HIV-2 RNA were analysed. An automated extraction machine (NucliSENSE easyMAG system, BioMedirux) was used for extraction of RNA from the plasma for analysis of HIV-2 RNA. The results are shown in Figure 12.

The assay detected 2.4 copies/ml of HIV-2 RNA in Patient B and 0.1 copies/ml in Patent A.

### Example 7 Evaluation of HIV-2 transcription

The same strategy used for HIV-1 transcription analysis was applied to HIV-2 transcription analysis. A single subject sample was available. The methodology for HIV-2 analysis is as described earlier herein.

The results are shown in Figure 13. The subject's HIV-2 plasma viral load was less than the detection level (Figure 13A). However, when the HIV-2 intracellular RNA transcript was detected and this RNA copy number is normalised to HIV-2 DNA copy number (Figure 13B), a relatively higher intracellular HIV-2 RNA level was observed.

### Example 8 Comparison of PCR assay sensitivity for HIV-1 DNA detection

The inventor developed two different PCR assay formats for detection of HIV-1 DNA which are compared below.

Real-time PCR assay was performed on DNA extracted from PBMCs using HIV-1 forward and reverse primers and Taqman probe as described in the Methods and Table 1 (SEQ ID NO: 6). End-point assay was performed using a biotin labelled HIV-1 forward primer, unlabelled reverse primer and digoxigenin-labelled probe (SEQ ID NO: 45). The sequence of the probe used for the end-point assay was a shorter version of the same probe that was used for real-time PCR.

The samples analysed for PCR were as follows:
Digoxigenin probe: Dig/-TTTTTTTTTTTTTTTGGAACCCACTGCTTA (SEQ ID. NO:45)
HIV-1 plasmid standard: 0, 4, 40, 400, 4,000, 40,000 copies of HIV-1 plasmid.
HUT-78 and OM10.1 mixed samples: 0, 4, 40, 400, 4000, 40,000 HIV-1 cells per 1 million (1 × 10⁶ uninfected cells)

HUT-78 cells are a CD4+ T cell line (not infected with HIV-1). OM10.1 cells are a CD4+ HIV-1 latently infected cell line in which each cell contains a single integrated HIV-1 provirus. To prepare "HIV-1 spiked" sample, the HUT-78 and OM10.1 cells were mixed together so that 0, 4, 40, 400, 4000, 40,000 OM10.1 cells were mixed with 1 × 10⁶ HUT-78 cells. This is designed to mimic an actual HIV-1 infected clinical sample from a subject.

Serial dilutions of plasmid standard were prepared as shown above and 8µl of standard was used in a 50µl PCR reaction.

Real-time PCR analysis and end-point PCR analysis of DNA extracted from the HUT-78/OM10.1 cell mixture was performed with 50 PCR cycles, against the HIV-1 plasmid standard. Results are shown in Table 5 below. 10 µl of PCR product was used for the endpoint assay analysis.

**Table 5: Comparison of Real-time PCR and Endpoint PCR assays**

| **Sample** | **Copies (copy/µl)** | **Cp (Real-time)** | **Real-Time PCR (copies of HIV-1/10⁶ cells)** | **End-point PCR (RLU/well)** |
|---|---|---|---|---|
| HIV-1 Standard | 0 | | | 0 |
| | 4 (newly made) | 38.29 | 7.31 | 18,760 |
| | 4 | 39.51 | 3.99 | 18,920 |
| | 40 | 35.14 | 40.30 | 18,990 |
| | 400 | 31.34 | 409.00 | 19, 110 |
| | 4000 | 27.87 | 3840.00 | 19,120 |
| | 40,000 | 24.22 | 40,800.00 | |
| | | | | |
| HUT78/OM10.1 | 0 | | 0 | |
| | 4 | ND | ND | 18,680 |
| | 40 | 39.31 | 4.40 | 18,860 |
| | 400 | 35.58 | 31.30 | 19,580 |
| | 4000 | 32.05 | 259.00 | 19,090 |
| | 40,000 | 28.46 | 2620.00 | 19,150 |
| | | | | |

The results shown are an average of duplicate assays.

Using real-time PCR, between 4 and 40,000 copies of HIV-1 plasmid standard was detected with a linear standard curve produced (not shown). As shown in Table 5, the minimum detection limit of the HUT-78/OM10.1 cell mixture was 40 OM10.1 cells in 1 × 10⁶ HUT-78 cells.

Using 40 cycles of end-point PCR, between 4 and 40,000 copies of HIV-1 plasmid standard was clearly detected . As shown in Table 5, the minimum detection limit of the HUT-78/OM10.1 cell mixture was 4 OM10.1 cells in 1 × 10⁶ HUT-78 cells. A luminescence reading of >1,000 RLU was considered positive detection.

The results show that both PCR assays provided very sensitive detection of HIV-1.

### Example 9 Comparison of specificity of PCR assays

Detection of HIV-2 DNA was measured using a HIV-2 plasmid standard and the results compared with the plasmid standard for HIV-1 to show specificity. HIV-1 and HIV-2 plasmid standards containing 0, 4, 40, 400, 4,000 and 40,000 copies were amplified with 50 PCR cycles by both real-time PCR and end-point PCR assays.

For real-time PCR, HIV-1 DNA was amplified with forward primer (SEQ ID NO:7) and reverse primer (SEQ ID NO:9). The amplicons were probed with a Taqman probe (SEQ ID NO:6) and copy number determined.

As shown in Table 6 below, the HIV-1 probe was able to detect from 4 to 40000 copies of HIV-1 DNA and was specific for HIV-1 since only HIV-1 DNA was amplified. Further, HIV-2 DNA was amplified with forward primer (SEQ ID NO:35) and reverse primer (SEQ ID NO:36). As shown in Table 6, the HIV-2 Taqman probe (SEQ ID NO:44) was able to detect from 4 to 40000 copies of HIV-2 and was specific for HIV-2 since none of the HIV-1 amplicons were detected with the HIV-2 probe.

**Table 6 Real-time assay of HIV-1 and HIV-2 standards**

| **Copies/µl** | **HIV-1 probe** | | **HIV-2 probe** | |
|---|---|---|---|---|
| | **HIV-1 (copies/µl)** | **HIV-2 (copies/µl)** | **HIV-1 (copies/µl)** | **HIV-2 (copies/µl)** |
| 0 | 0 | 0 | 0 | 0 |
| 4 | 4.7 | 0 | 0 | 4.7 |
| 40 | 37.2 | 0 | 0 | 51.6 |
| 400 | 524.0 | 0 | 0 | 364.0 |
| 4000 | 4000.0 | 0 | 0 | 2260.0 |
| 40000 | 40000.0 | 0 | 0 | 60400.0 |

For end-point PCR, HIV-1 DNA was amplified with a biotin labelled forward primer (SEQ ID NO:7) and an unlabelled reverse primer (SEQ ID NO:9). The amplicons were probed with a digoxigenin (Dig) labelled HIV-1 probe (SEQ ID NO:45). The results are shown in Table 7 below.

HIV-2 DNA was amplified with an unlabelled forward primer (SEQ ID NO:35) and a biotin labelled reverse primer (SEQ ID NO:36). The amplicons were probed with a digoxigenin (Dig) labelled HIV-2 probe (SEQ ID NO:49).
Dig HIV-1: Dig/-TTTTTTTTTTTTTTTGGAACCCACTGCTTA (SEQ ID NO: 45)
Dig HIV-2: Dig/-TTTTTTTTTTTTTTTCCAGCAGTAGCAGGT (SEQ ID NO: 49)

As shown in Table 7, the HIV-1 probe was able to detect from 4 to 40000 copies of HIV-1 and was specific for HIV-1. The HIV-2 probe was also able to detect from 4 to 40000 copies of HIV-2 and was specific for HIV-2.

**Table 7 End-point PCR assay of HIV-1 and HIV-2 standards**

| **Copies/µl** | **HIV-1 probe** | | **HIV-2 probe** | |
|---|---|---|---|---|
| | **HIV-1 (copies/µl)** | **HIV-2 (copies/µl)** | **HIV-1 (copies/µl)** | **HIV-2 (copies/µl)** |
| 0 | 0 | 0 | 0 | 0 |
| 4 | 40,280 | 0 | 0 | 62,890 |
| 40 | 48,200 | 0 | 0 | 52,110 |
| 400 | 65,260 | 0 | 0 | 48,910 |
| 4000 | 70,360 | 0 | 0 | 43,280 |
| 40000 | 73,088 | 0 | 0 | 42,070 |

The results shown are an average of duplicate assays.

These results show that both real-time PCR and end-point PCR assays provide specific detection of HIV-1 or HIV-2 with no cross-reactivity.

### Example 10 Comparison of real-time and end-point PCR assays on CD14+ isolated cells

The assay described in Example 8 was repeated. However, in the experiment the HIV-1 plasma standard was diluted further to provide 0.2 copies/µl and 0.4 copies/µl. The number of PCR cycles for end-point PCR was also reduced from 50 to 40.

Blood samples were obtained from HIV-1 infected subjects and their white blood cells were separated into CD14 positive (CD14+) and CD14 negative (CD14-) cells. CD14+ cells comprise approximately one third of white blood cells and are predominantly a monocyte population. These cells differentiate into macrophages in the peripheral blood and into microglial cells in the brain. CD14- cells comprise approximately two thirds of white blood cells and include CD4+ T cells.

6ml blood samples were obtained from the subjects for separation into CD14+ and CD14- cell populations. The subjects fell into two categories, those that had treatment success with anti-viral therapy and those with treatment failure. Treatment success subjects were defined as those whose HIV-1 plasma levels were under control with anti-retroviral therapy (ART). Their HIV-1 viral load was consistently under detection levels for over 2 years. Treatment failure subjects were defined as those whose HIV-1 plasma viral load was detectable (i.e. between 20 and 400 copies/ml in plasma) over 2 years.

HIV-1 DNA was amplified by end-point PCR for 40 cycles using forward primer (SEQ ID NO:7) and reverse primer (SEQ ID NO:9). HIV-1 DNA was amplified by real-time PCR for 50 cycles using forward primer (SEQ ID NO:7) and reverse primer (SEQ ID NO:9).

Serial dilutions of plasmid standard were prepared as shown above and 8µl of standard was used in a 50µl PCR reaction.

The results are shown in Table 8.

**Table 8 Real-time and End-point PCR assay of separated CD14+ and CD14- cells from blood**

| **HIV-1 Standard (copies/µl)** | | **End-point assay Luminescence (RLU) 40 cycles** | **Real-time assay 50 cycles (Cp cycle)** |
|---|---|---|---|
| 0 | | 0 | 0 |
| 0.2 | | 0 | 0 |
| 0.4 | | 54800 | 0 |
| 4.0 | | 63560 | 4.3 (34.01) |
| 40.0 | | 64460 | 38.2 (30.83) |
| 400.0 | | 66340 | 376.0 (27.52) |
| | | | |

| **Subject ID** | **Cell type** | | |
|---|---|---|---|
| 307^ | CD14+ | 0 | 0 |
| | CD14- | 18,520 | 0.37 (45.00) |
| 308# | CD14+ | 19,870 | 0.37 (45.00) |
| | CD14- | 18,510 | 5.41 (38.89) |
| 309# | CD14+ | 41,470 | 0.47 (44.38) |
| | CD14- | 63,220 | 1.580 (41.47) |
| 318^ | CD14+ | 0 | 0 |
| | CD14- | 38,560 | 0.059 (40.02) |
| 319^ | CD14+ | 0 | 0 |
| | CD14- | 20,100 | 0 |
| 322# | CD14+ | 57,830 | 0 |
| | CD14- | 70,190 | 0.007 (43.36) |
| 323# | CD14+ | 0 | 0 |
| | CD14- | 64,410 | 0.001 (45.77) |
| 324# | CD14+ | 54,390 | 0 |
| | CD14- | 60,490 | 0.009 (42.90) |

Treatment success subjects are indicated by ^. Treatment failure subjects are indicated by #. The results shown are an average of duplicate assays.

As shown in Table 8, the detection limit of real-time PCR was 4 copies/µl) of HIV-1 DNA standard. The end-point assay was able to detect 0.4 copies/µl of HIV-1 DNA and thus appeared to be ten times more sensitive than the real-time PCR assay.

Real-time PCR analysis showed HIV-1 detection in most CD14- cell populations from the subjects. However, the assay did not detect HIV-1 in CD14+ cell populations from subjects with treatment failure. In contrast, the end point assay was able to detect HIV-1 DNA in all CD14- cell populations (whether treatment positive or treatment negative) and in CD14+ cell populations from treatment failure subjects suggesting that the end-point assay provides a more sensitive detection method.

### Example 11 Analysis of HIV-2 clinical sample

In this example, the inventor wanted to determine whether plasma HIV-2 levels would elevate (i.e. rebound) after ceasing ART. The present example shows a subject with latent HIV-2 infection under ART treatment.

The present example relates to a subject whose HIV-2 plasma viral load (pVL; i.e. HIV-2 copy number in plasma) was consistently suppressed due to her ART treatment. Over the two years the subject had been monitored, her pVL was below the detection limit. The subject, against doctor's advice stopped taking her ART in mid-June 2017. A blood sample obtained from the subject was obtained a month after stopping ART.

Plasma was extracted from the subject's sample and the presence of HIV-2 RNA determined using the forward primer (SEQ ID NO:35) and reverse primer (SEQ ID NO:36) and probe (SEQ ID NO:37). End-point and real-time PCR amplification were performed with 50 cycles of PCR.

The subject's sample was compared with (i) an HIV-2 plasmid standard (2 to 20,000 copies of HIV-2 plasmid DNA) (ii) an HIV-2 positive control sample (iii) two HIV-2 RNA spiked controls which were prepared by spiking HIV-2 RNA (released into the culture supernatant from HIV-2 infected CD4+ cells) at two different concentrations (one high and one low) into normal plasma and (iv) a negative control (negative plasma sample).

The result of real-time PCR analysis and end-point PCR analysis is shown in Table 9 below.

**Table 9 PCR assay of HIV-2 clinical sample**

| **Sample** | **HIV-2 Standard** | **End-point assay (CPU) with HIV-2 probe** | **Real-time assay copies/ml (Cp) with HIV-2 probe** |
|---|---|---|---|
| | 0 | 0 | 0 |
| | 2 | 29510 | 2 (44.80) |
| | 20 | 33090 | 20 (40.03) |
| | 200 | 35070 | 200 (35.82) |
| | 2000 | 35750 | 2000 (31.88) |
| | 20000 | 36390 | 20000 (27.21) |
| | | | |
| Subject | | 29,290 | 4.94 (42.67) |
| Spiked control #1 | | 29,880 | 193.00 (35.92) |
| Spiked control #2 | | 28,700 | 2.74 (43.76) |
| Positive control | | 34590 | 147.00 (36.42) |
| Negative control | | 0 | 0 |

As shown in Table 9, real-time PCR was able to detect the presence of HIV-2 in the subject's sample as well as in the spiked and positive controls. Accordingly, the assay confirmed that the subject still had HIV-2 in her blood.

Additionally, end-point PCR detected the presence of HIV-2 in the subject's sample as well as in the spiked and positive controls. Accordingly, the assay also confirmed that the subject still had HIV-2 in her blood.

### Example 12 Analysis of HIV-1 clinical samples

Two subject case studies are presented in this example. The first subject (subject 320) had high HIV-1 antibody titre. Western blot analysis showed HIV-1 antibodies in the subject's plasma against all HIV-1 (env, gag, pol). Based on serology, the subject had an established HIV-1 infection, however her pVL was negative. She was not receiving any ART.

With regard to the second subject (subject 321), Western blot analysis was inconclusive and had been for several years. The subject's pVL was negative and he was not receiving any ART. Based on serology, the subject was considered to be likely HIV-1 negative, however a more sensitive assay was required to determine whether the subject was conclusively HIV-1 negative.

PBMCs were isolated from whole blood as described in the methods and DNA extracted. For assay validation, the samples were run with the following controls: (i) an HIV-1 plasmid standard containing 0, 0.2, 0.4, 4, 40 and 400 copies/µl, (ii) a positive control containing 40 OM10.1 cells in 1 × 10⁶ HUT78 cells; (iii) a further positive control being a sample from a known HIV-1 positive subject (subject 291) and (iv) the HUT-78 cell line as a negative control. The assay was considered valid when the assay detected 40 OM10.1 cells.

Real-time PCR was conducted in duplicate using the forward primer (SEQ ID NO:7) and reverse primer (SEQ ID NO:9) and HIV-1 probe (SEQ ID NO:6). End-point PCR was also conducted in duplicate using a biotin labelled reverse primer (SEQ ID NO:9), an unlabelled forward primer (SEQ ID NO:7) and a digoxygenin labelled HIV-1 probe (SEQ ID NO:45). Both Real-Time PCR and End-Point PCR were conducted with 50 cycles with the same run.

The analysis of the subjects is shown in Table 10 below.

**Table 10 PCR assay of HIV-1 clinical samples**

| **Sample** | **HIV-1 standard** | **End-point assay (CPU) with HIV-1 probe** | **Real-time assay copies/ml (Cp ) with HIV-1 probe** |
|---|---|---|---|
| | 0 | 0 | 0 |
| | 0.2 | 71053 | 0 |
| | 0.4 | 65448 | 0.40 (44.30) |
| | 4.0 | 66840 | 2.52 (37.93) |
| | 40.0 | 78905 | 25.00 (34.63) |
| | 400.0 | | 254.00 (31.30) |
| | | | |
| Subject 320 | | 8610 | 0.43 (41.98) |
| | | 7520 | 0.00 |
| Subject 321 | | 0 | 0.00 |
| | | 0 | 0.00 |
| Negative control | | 0 | 0.00 |
| Positive control | | 10450 | 1.48 (38.76) |
| Positive control (subject 291) | | 9100 | 0.89 (39.71) |

With regard to the real-time assay, subject 320 showed one positive result and one negative result suggesting that the HIV-1 level was just on the detection limit for this assay. The estimated HIV-1 copy number for this subject is 27 HIV-1 infected cells per 10⁶ PBMCs (0.0027%). Subject 321 was clearly negative for HIV-1.

With regard to the end-point assay, subject 320 was clearly positive and subject 321 was clearly negative.

### Example 13 Analysis of HIV-1 in mother and newborn

This example describes the analysis of a mother (subject 118) and her 2 month baby (subject 142). The mother had previously been diagnosed as HIV-1 positive, however when tested by Xpert^{®} assay (Cepheid) and standard viral load assay, HIV-1 could not be detected. A more sensitive assay was required to definitely confirm the HIV status of both the mother and baby.

Quantification of HIV-1 DNA and RNA was performed by real-time PCR according to the methods described in the disclosure. DNA and RNA respectively were extracted from 2ml of whole blood (4ml whole blood total) in which PBMCs had been isolated using standard ficoll-hypaque density gradient centrifugation. The sample obtained from subject 118 was run in duplicate. The sample obtained from subject 142 was run four times.

The copy number determined by each assay is shown below:

**Table 11 HIV-1 copy number**

| **Subject** | **HIV-1 copies / 10⁶ cells** | **HIV status** |
|---|---|---|
| **HIV-1 DNA assay** | | |
| Subject 118 (mother) | 1.82 | Positive |
| Subject 142 (baby) | 0 | Negative |
| Negative control | 0 | Negative |
| Positive control (subject 95) | 34 | Positive |
| Positive control (subject 97) | 1410 | Positive |

| **Transcriptional HIV-1 RNA assay** | | |
|---|---|---|
| Subject 118 (mother) | 51 | Positive |
| Negative control | 0 | Negative |
| Positive control (subject 95) | 598 | Positive |

Subject 95 was a HIV-1 positive individual with pVL less than 20 copies/ml and CD4+ count 588.
Subject 97 was a HIV-1 positive individual with immunological failure; VL suppressed below 20 copies/ml and low CD4 count.

Accordingly, the assay could definitively diagnose the mother as HIV-1 positive and the baby as HIV-1 negative. T/A cloning confirmed that the amplified sequence was R region sequence of HIV-1. Sequence data demonstrated that the HIV-1 Taqman probe assay detected HIV-1 R region sequence extracted from the samples.

### Example 14 Analysis of sample determined to be negative by GeneXpart and viral load.

This example describes quantification of HIV-1 DNA in a subject (subject 197) who had previously been found to be HIV-1 negative by GeneXpart assay and viral load assay.

Quantification of HIV-1 DNA was performed by real-time PCR (in triplicate) according to the methods described in the disclosure. The results are summarised in Table 12.

**Table 12 HIV-1 copy number**

| **Subject** | **HIV-1 copies/10⁶ cells** | **HIV status** |
|---|---|---|
| Subject 197 | 108 | Positive |
| Negative control (Subject 94) | 0 | Negative |
| Positive control (Subject 124) | 983 | Positive |

| | | |
|---|---|---|
| Subject 124 is a HIV-1 positive control with immunological failure; VL suppressed below 20 copies/ml and low CD4 count. DNA was extracted from the sample with lysis buffer. | | |

Accordingly, the assay could definitively diagnose this subject as HIV-1 positive. T/A cloning confirmed that the amplified sequence was R region sequence of HIV-1. Sequence data demonstrated that the HIV-1 Taqman probe assay detected HIV-1 R region sequence extracted from the samples.

### Example 15 HIV-1 Transcription Assay

Automated DNA and RNA extraction from 26 subject samples obtained from St Vincent's Hospital was prepared using a Maxwell extraction machine (Promega). Quantification of DNA and RNA was determined according to the protocols described herein. The medical records of the 28 subjects were examined and the subjects categorised into two groups: the first being the "optimal ART" group (n=7) and the second being the "sub-optimal ART" group (n=21). The optimal ART group were those subjects whose pVL was consistently suppressed for at least 6 months and whose CD4+ T cell count was over 500 counts. The "sub-optimal ART" group (n=21) experienced blips (occasional and transient elevation of pVLs within a 6 month period) and immunological failure (CD4+ T cell counts less than 500 counts/µl).

As shown in Figure 14A, plasma VL did not differ between the two groups of subjects (ie. optimal ART and sub-optimal ART groups) and their levels were less than the detection limit of the assay.

When HIV-1 DNA levels were examined (Figure 14B), there was some difference observed between the two groups. This was more pronounced when RNA levels (transcriptional activity) was examined (Figure 14C). Quantification was determined and normalised as described herein.

As shown in Figure 15, a further group within the "optimal ART" group was identified, which is referred to as "Improved-optimal ART". This group is defined as subjects whose HIV-1 DNA is less than 400 copies per 1 × 10⁶ cells. This group demonstrated very little ongoing RNA expression likely due to transcriptional suppression. Accordingly, HIV-1 integration into the genome is reduced. Ideally, the goal of ART is to have subjects in the improved optimal ART category.

The data shown thus provides a way of assessing HIV RNA transcriptional activity in a subject, particularly when that subject is receiving ART.

### Example 16 Evidence of HIV-1 activation in HIV-1 latently infected cells

It is known that HIV-1 can be activated from latently infected cell reservoirs when exposed to HIV-1 stimuli. If latently infected cell reservoirs are being activated, it would be expected that transcription levels would increase. The present example examined this hypothesis by measuring RNA transcription following exposure to various stimuli.

Fourteen (14) subjects who were being successfully treated by ART were examined. These subjects had pVL levels which were under the limit of detection and CD4+ T cell count greater than 500 cells/µl.

PBMCs were obtained from each subject (between 3-6ml) and divided into four equal portions (preferably each portion containing > 1 × 10⁶ cells). If cell numbers were not sufficient enough for a given subject then the PBMCs were divided into three portions.

The groups were designated as follows:
Group 1: BI (BI-2536); Polo-like kinase (PLK) inhibitor
Group 2: JQ1; Bromodomain inhibitor
Group 3: PMA; Phorbol-12-myristate-13-acetate (HIV activator)
Group 4: control (no stimuli)

PBMCs were cultured in RPMI medium with 20% FBC in the presence of the indicated agent for 14 hours in a 24 well plate in a CO₂ incubator. After 14 hours total RNA was extracted from the cultured PBMCs. Analysis of HIV-RNA was performed according to the methods described herein.

As shown in Figure 16, in the presence of activating stimuli, HIV-1 transcription was stimulated and this increased transcription could be detected although the degree of activation varied from subject to subject suggesting that the mechanisms of HIV-1 latency are diverse and may require more than one mechanism to transition from a latent state to an active state. Accordingly, this assays allow detection of HIV in latently infected cells which pVLs are typically under detection limits in patient plasma VL. The data confirmed that the developed assays detecting transcriptional activities are a true signal, since levels of the transcriptional actives were elevated in the presence of HIV-1 stimuli in the culture medium, compared with control culture (without presence of stimuli).

### Example 16 Quantification of HIV by end-point assay

This example shows quantification of HIV-1 by end-point PCR. Rather than performing 50 cycles of amplification, the number of PCR cycles were reduced to 40 cycles. Samples from eight (8) subjects were examined. Briefly, DNA from PBMCs obtained from each subject was extracted using a Maxwell Extraction System (Promega) followed by 40 cycle PCR amplification. Amplification was carried out using forward primer (SEQ ID NO:7) and reverse primer (SEQ ID NO:9) and probe (SEQ ID NO:6). The PCR curve generated from the plasmid standards is shown in Figure 17 and the estimated copy number was determined from this standard curve.

The results are shown in Table 13 below.

**Table 13 Quantitation of end-point PCR amplified products**

| **HIV-1 Plasmid standard Copies** | **End point PCR (RLU)** | **Copies/µl** |
|---|---|---|
| 0 | 0 | |
| 0.2 | 50 | |
| 0.4 | 5140 | |
| 4.0 | 12970 | |
| 40.0 | 17820 | |
| 400.0 | 23780 | |
| | | |

| **Subjects** | | |
|---|---|---|
| A | 14120 | 10.05 |
| B | 18790 | 49.04 |
| C | 10360 | 2.81 |
| D | 12360 | 5.53 |
| E | 15870 | 18.21 |
| F | 16640 | 23.64 |
| G | 16400 | 21.79 |
| H | 16200 | 20.36 |

### Example 17 PCR assays of dried blood spot testing (DBS) biosampling

Real-time and end-point PCR analysis was undertaken on dried blood spot (DBS) samples. For this analysis, eight samples having various HIV-1 RNA copy numbers (pVL) were amplified by real-time and end-point PCR. From those samples, 70µl of whole blood was spotted onto DBS paper and RNA extracted from those spotted samples.

As shown in Table 14, HIV-1 RNA could be detected in the samples using both real-time and end-point PCR assay methods. For real-time PCR, amplification was carried out using forward primer (SEQ ID NO:7) and reverse primer (SEQ ID NO:9) and probe (SEQ ID NO:6). For end-point PCR, amplification was carried out using an unlabelled forward primer (SEQ ID NO:7) and biotin labelled reverse primer (SEQ ID NO:9) and Dig labelled probe (SEQ ID NO:45).

These results show that samples can be collected from remote areas or in areas that are under developed or under resourced and by transferred to a site where the analysis can be conducted.

**Table 14 PCR analysis of DBS**

| **HIV-1 RNA standard** | **End-point assay (RLU) HIV-1 probe** | **Real-time assay HIV-1 probe (Cp cycle)** |
|---|---|---|
| 0 | 0 | |
| 4 | 27840 | 4 (39.0) |
| 40 | 28720 | 40 (35.55) |
| 400 | 27140 | 400 (30.94) |
| | | |

| **Subject (copies HIV-1 RNA/ml)** | | |
|---|---|---|
| 1 (2663) | 22690 | 14.7 (36.92) |
| 2 (10548) | 23210 | 56.1 (34.57) |
| 3 (158000) | 23010 | 475.7 (30.83) |
| 4 (7901) | 23080 | 9.0 (37.78) |
| 5 (31005) | 23400 | 12.6 (37.19) |
| 6 (42000) | 22730 | 0.2(44.94) |
| 7 (3,870) | 23550 | 50.1 (34.77) |
| 8 (negative control) | 0 | |

## Claims

1. A method of detecting human immunodeficiency virus (HIV) in a subject with HIV-1 or HIV-2, or a subject suspected of having an HIV infection (AIDS), the method comprising performing PCR amplification of R region nucleic acid in a biological sample obtained from the subject, wherein the amplification comprises forward and reverse primers which hybridise to sequences located within the R region of the long terminal repeats (LTRs) of HIV, and subsequently detecting any amplification, wherein detecting amplification is indicative of the presence of HIV in the subject, wherein the amplification will result in both R regions present within the 5' and 3' LTRs to be amplified, and wherein:
(i) the forward primer is a HIV-1 primer consisting of the sequence SEQ ID NO:7, or SEQ ID NO:29; and the reverse primer is a HIV-1 primer consisting of the sequence SEQ ID NO:9, or SEQ ID NO:30; or
(ii) the forward primer is a HIV-2 primer consisting of the sequence SEQ ID NO:14, SEQ ID NO:33, or SEQ ID NO:35; and the reverse primer is a HIV-2 primer consisting of the sequence SEQ ID NO:16, SEQ ID NO:32, SEQ ID NO:34 or SEQ ID NO:36.

2. The method according to claim 1, wherein detecting amplification is performed with a labelled oligonucleotide probe which hybridises to a sequence within the amplified R region sequence.

3. The method according to any one of claims 1 to 2, wherein the nucleic acid is DNA or reverse transcribed RNA (cDNA).

4. The method of any one of claims 1 to 3, wherein the method comprises:
(i) quantifying HIV DNA copy number in a biological sample from the subject with HIV, or suspected of having an HIV infection, the method comprising quantifying the amplified HIV-R region sequence by reference to a corresponding HIV plasmid standard to obtain the copy number of HIV DNA per volume of sample; or
(ii) quantifying HIV RNA copy number in a biological sample from the subject with HIV, or suspected of having an HIV infection, the method comprising quantifying the amplified HIV-R region sequence by reference to a corresponding HIV plasmid standard to obtain the copy number of HIV RNA per volume of sample.

5. The method according to any one of claims 1 to 4, wherein the amplification is real-time PCR or end-point PCR.

6. The method according to claim 4 or 5, further comprising:
(i) normalising the HIV DNA copy number against a DNA standard to obtain the HIV DNA copy number per cell(s) in the biological sample; or
(ii) normalising the HIV RNA copy number against a RNA standard to obtain the HIV RNA copy number per cell(s) in the biological sample.

7. The method according to any one of claims 4 to 6, comprising:
(i) obtaining an aliquot of the sample wherein the DNA or RNA has been extracted;
(ii) contacting the aliquot with a labelled hydrolysis oligonucleotide probe which hybridises to R region sequence of a long terminal repeat (LTR) of the HIV DNA or of the reverse transcribed HIV RNA (cDNA);
(iii) contacting the aliquot with forward and reverse primers which hybridise to sequences within the R region sequence of the HIV DNA or of the reverse transcribed HIV RNA (cDNA);
(iv) amplifying the R region sequence by PCR;
(v) extrapolating the signal obtained from the labelled oligonucleotide to a standard curve obtained by corresponding amplifications of serial dilutions of an HIV standard to derive the HIV-R region DNA or RNA copy number per volume of DNA or RNA in the aliquot.

8. The method according to any one of claims 1 to 4, wherein the forward or reverse primer is labelled with biotin and the probe is a labelled with digoxigenin (Dig).

9. The method according to any one of claims 1 to 8, wherein:
(i) the forward primer is SEQ ID NO:7 and the reverse primer is SEQ ID NO:9;
(ii) the forward primer is SEQ ID NO:14 and the reverse primer is SEQ ID NO:16;
(iii) the forward primer is SEQ ID NO:33 and the reverse primer is SEQ ID NO:34;
(iv) the forward primer is SEQ ID NO:35 and the reverse primer is SEQ ID NO:36.

10. A method for monitoring anti-retroviral therapy (ART) being administered to an HIV positive subject, comprising quantifying the HIV DNA copy number according to any one of claims 4 to 8 over at least two time points and comparing the difference in HIV R region DNA copy number between the at least two time points wherein a decrease in the HIV R region DNA copy number indicates the subject is receiving optimal/effective ART.

11. A method for assessing the effectiveness of anti-retroviral therapy (ART) administered to an HIV positive subject, the method comprising:
(i) quantifying the HIV-R region DNA copy number according to any one of claims 4 to 7;
(ii) quantifying the HIV-R region RNA copy number according to any one of claims 4 to 7;
(iii) determining a normalised HIV RNA copy number in the sample by dividing the value obtained in step (i) with that obtained in step (ii); and
(iv) comparing the normalised HIV RNA copy number value to one or more previous normalised values obtained from the same subject;
wherein a decrease in the normalised HIV RNA copy number indicates that the subject is receiving optimal/effective ART.

12. The method according to claim 11 further comprising preparing DNA and RNA from a biological sample that has been obtained from the subject, wherein the biological sample is a population of cells selected from blood or tissue or any other biological fluid in which HIV-infected cells are present, for example the biological sample is PBMCs.

13. The method according to any preceding claim, wherein the R region sequence consists of the sequence set forth in SEQ ID NO:1 or SEQ ID NO:2.

14. The method according to any one of claims 2 to 13, wherein:
(i) the oligonucleotide probe:
(a) binds to a sequence comprising or consisting of the sequence 5' TAAGCAGTGGGTTCCCT 3' (SEQ ID NO:3) or a sequence at least 90% identical thereto; or
(b) comprises or consists of the sequence SEQ ID NO:4, SEQ ID NO:5, SEQ ID NO:6, SEQ ID NO:45, SEQ ID NO:46, SEQ ID NO:47, or SEQ ID NO:48; and
(ii) the forward primer is an HIV-1 primer consisting of the sequence SEQ ID NO:7, or SEQ ID NO:29; and
(iii) the reverse primer is an HIV-1 primer consisting of the sequence SEQ ID NO:9, or SEQ ID NO:30.

15. The method according to any preceding claim, wherein:
(i) the oligonucleotide probe:
(a) binds to a sequence comprising or consisting of the sequence 5' GCCTGGGTGTTCCCTGCTAGACTCT 3' (SEQ ID NO:11) or a sequence at least 90% identical thereto; or
(b) comprises or consists of the sequence SEQ ID NO:12, SEQ ID NO:13, SEQ ID NO: 37, SEQ ID NO: 38, SEQ ID NO:39, or SEQ ID NO:40, SEQ ID:41, SEQ ID:42, SEQ ID:43, SEQ ID:44, SEQ ID NO:49, SEQ ID NO:50, SEQ ID NO:51, SEQ ID NO:52 or SEQ ID NO:53; and
(ii) the HIV-2 forward primer consists of the sequence according to SEQ ID NO:14, SEQ ID NO:33, or SEQ ID NO:35; and
(iii) the HIV-2 reverse primer consists of the sequence SEQ ID NO:16, SEQ ID NO:32, SEQ ID NO:34 or SEQ ID NO:36.

16. A composition comprising a labelled oligonucleotide probe and a forward and reverse primer selected from a combination of one or more of the following:
(i) oligonucleotide probe comprising or consisting of SEQ ID NO:4, SEQ ID NO:5, SEQ ID NO:6, SEQ ID NO:45, SEQ ID NO:46, SEQ ID NO:47, or SEQ ID NO:48;
(ii) forward primer consisting of SEQ ID NO:7 or SEQ ID NO:29; and
(iii) reverse primer consisting of SEQ ID NO:9 or SEQ ID NO:30.

17. A composition comprising a labelled oligonucleotide probe and a forward and reverse primer selected from a combination of one or more of the following:
(i) oligonucleotide probe comprising or consisting of SEQ ID NO:12, SEQ ID NO:13, SEQ ID NO: 37, SEQ ID NO: 38, SEQ ID NO:39, SEQ ID NO:40; SEQ ID NO:41, SEQ ID NO:42, SEQ ID NO:43, SEQ ID NO:44, SEQ ID NO:49, SEQ ID NO:50, SEQ ID NO:51, SEQ ID NO:52 or SEQ ID NO:53;
(ii) forward primer consisting of SEQ ID NO:14, SEQ ID NO:33, or SEQ ID NO:35;
(iii) reverse primer consisting of SEQ ID NO:16, SEQ ID NO:32, SEQ ID NO:34 or SEQ ID NO:36.

18. A kit for detecting HIV-1, comprising the composition comprising the labelled oligonucleotide probe, forward and reverse primer of claim 16, together with suitable reagents and instructions for detecting and quantifying HIV-1.

19. A kit for detecting HIV-2, comprising the composition comprising the labelled oligonucleotide probe, forward and reverse primer of claim 17, together with suitable reagents and instructions for detecting and quantifying HIV-2.

## Patentansprüche

1. Verfahren zum Nachweisen von menschlichem Immunschwächevirus (Human Immunodeficiency Virus, HIV) bei einem Individuum mit HIV-1 oder HIV-2 oder bei einem Individuum, bei dem eine HIV-Infektion (AIDS) vermutet wird, wobei das Verfahren Durchführen einer PCR-Amplifikation von R-Region-Nukleinsäure in einer von dem Individuum erhaltenen biologischen Probe, wobei die Amplifikation Vorwärts- und Rückwärtsprimer umfasst, die an innerhalb der R-Region der LTRs (Long Terminal Repeats) von HIV liegende Sequenzen hybridisieren, und anschließendes Nachweisen jeglicher Amplifikation umfasst, wobei Nachweisen von Amplifikation das Vorliegen von HIV bei dem Individuum anzeigt, wobei die Amplifikation dazu führt, dass beide innerhalb der 5'- und 3'-LTRs vorhandenen R-Regionen amplifiziert werden, und wobei es sich:
(i) bei dem Vorwärtsprimer um einen HIV-1-Primer handelt, der aus der Sequenz SEQ ID NO:7 oder SEQ ID NO:29 besteht; und bei dem Rückwärtsprimer um einen HIV-1-Primer handelt, der aus der Sequenz SEQ ID NO:9 oder SEQ ID NO:30 besteht; oder
(ii) bei dem Vorwärtsprimer um einen HIV-2-Primer handelt, der aus der Sequenz SEQ ID NO:14, SEQ ID NO:33 oder SEQ ID NO:35 besteht; und bei dem Rückwärtsprimer um einen HIV-2-Primer handelt, der aus der Sequenz SEQ ID NO:16, SEQ ID NO:32, SEQ ID NO:34 oder SEQ ID NO:36 besteht.

2. Verfahren nach Anspruch 1, wobei Nachweisen von Amplifikation mit einer markierten Oligonukleotidsonde erfolgt, die an eine Sequenz innerhalb der amplifizierten R-Region-Sequenz hybridisiert.

3. Verfahren nach einem der Ansprüche 1 bis 2, wobei es sich bei der Nukleinsäure um DNA oder revers transkribierte RNA (cDNA) handelt.

4. Verfahren gemäß einem der Ansprüche 1 bis 3, wobei das Verfahren Folgendes umfasst:
(i) Quantifizieren der HIV-DNA-Kopienzahl in einer biologischen Probe von dem Individuum, bei dem HIV vorliegt oder eine HIV-Infektion vermutet wird, wobei das Verfahren Quantifizieren der amplifizierten HIV-R-Region-Sequenz unter Bezugnahme auf einen entsprechenden HIV-Plasmid-Standard unter Erhalt der Kopienzahl von HIV-DNA pro Volumen Probe; oder
(ii) Quantifizieren der HIV-RNA-Kopienzahl in einer biologischen Probe von dem Individuum, bei dem HIV vorliegt oder eine HIV-Infektion vermutet wird, wobei das Verfahren Quantifizieren der amplifizierten HIV-R-Region-Sequenz unter Bezugnahme auf einen entsprechenden HIV-Plasmid-Standard unter Erhalt der Kopienzahl von HIV-RNA pro Volumen Probe.

5. Verfahren nach einem der Ansprüche 1 bis 4, wobei es sich bei der Amplifikation um Echtzeit-PCR oder Endpunkt-PCR handelt.

6. Verfahren nach Anspruch 4 oder 5, ferner umfassend:
(i) Normalisieren der HIV-DNA-Kopienzahl gegen einen DNA-Standard unter Erhalt der HIV-DNA-Kopienzahl pro Zelle(n) in der biologischen Probe; oder
(ii) Normalisieren der HIV-RNA-Kopienzahl gegen einen RNA-Standard unter Erhalt der HIV-RNA-Kopienzahl pro Zelle(n) in der biologischen Probe.

7. Verfahren nach einem der Ansprüche 4 bis 6, umfassend:
(i) Erhalten einer Portion der Probe, wobei die DNA oder RNA extrahiert wurde;
(ii) In-Kontakt-Bringen der Portion mit einer markierten Hydrolyse-Oligonukleotidsonde, die an R-Region-Sequenz einer LTR (Long Terminal Repeat) der HIV-DNA oder der revers-transkribierten HIV-RNA (cDNA) hybridisiert;
(iii) In-Kontakt-Bringen der Portion mit Vorwärts- und Rückwärtsprimern, die an Sequenzen innerhalb der R-Region-Sequenz der HIV-DNA oder der revers-transkribierten HIV-RNA (cDNA) hybridisieren;
(iv) Amplifizieren der R-Region-Sequenz mit PCR;
(v) Extrapolieren des von dem markierten Oligonukleotid erhaltenen Signals auf eine durch entsprechende Amplifikationen von Verdünnungsreihen eines HIV-Standards erhaltene Standardkurve zur Ableitung der to derive the HIV-R-Region-DNA- bzw. -RNA-Kopienzahl pro Volumen DNA bzw. RNA in der Portion.

8. Verfahren nach einem der Ansprüche 1 bis 4, wobei der Vorwärts- oder Rückwärtsprimer mit Biotin und die Sonde mit Digoxigenin (Dig) markiert ist.

9. Verfahren nach einem der Ansprüche 1 bis 8, wobei es sich bei:
(i) dem Vorwärtsprimer um SEQ ID NO:7 und dem Rückwärtsprimer um SEQ ID NO:9 handelt;
(ii) dem Vorwärtsprimer um SEQ ID NO:14 und dem Rückwärtsprimer um SEQ ID NO:16 handelt;
(iii) dem Vorwärtsprimer um SEQ ID NO:33 und dem Rückwärtsprimer um SEQ ID NO:34 handelt;
(i) dem Vorwärtsprimer um SEQ ID NO:35 und dem Rückwärtsprimer um SEQ ID NO:36 handelt.

10. Verfahren zur Überwachung einer einem HIV-positiven Individuum verabreichten antiretroviralen Therapie (ART), umfassend Quantifizieren der HIV-DNA-Kopienzahl nach einem der Ansprüche 4 bis 8 über wenigstens zwei Zeitpunkte und Vergleichen des Unterschieds der HIV-R-Region-DNA-Kopienzahl zwischen den wenigstens zwei Zeitpunkten, wobei eine Abnahme der HIV-R-Region-DNA-Kopienzahl anzeigt, dass das Individuum eine optimale/wirksame ART erhält.

11. Verfahren zur Beurteilung der Wirksamkeit einer einem HIV-positiven Individuum verabreichten antiretroviralen Therapie (ART), wobei das Verfahren Folgendes umfasst:
(i) Quantifizieren der HIV-R-Region-DNA-Kopienzahl nach einem der Ansprüche 4 bis 7;
(ii) Quantifizieren der HIV-R-Region-RNA-Kopienzahl nach einem der Ansprüche 4 bis 7;
(iii) Bestimmen einer normalisierten HIV-RNA-Kopienzahl in der Probe durch Dividieren des in Schritt (i) erhaltenen Werts mit dem in Schritt (ii) erhaltenen; und
(iv) Vergleichen des normalisierten HIV-RNA-Kopienzahlwerts mit einem oder mehreren vorherigen von dem gleichen Individuum erhaltenen normalisierten Werten;
wobei eine Abnahme der normalisierten HIV-RNA-Kopienzahl anzeigt, dass das Individuum eine optimale/wirksame ART erhält.

12. Verfahren nach Anspruch 11, ferner umfassend Präparieren von DNA und RNA aus einer biologischen Probe, die von dem Individuum erhalten wurde, wobei es sich bei der biologischen Probe um eine aus Blut oder Gewebe oder einer anderen biologischen Flüssigkeit, in der HIV-infizierte Zellen vorhanden sind, ausgewählte Population von Zellen, beispielsweise um PBMC handelt.

13. Verfahren nach einem vorhergehenden Anspruch, wobei die R-Region-Sequenz aus der Sequenz gemäß SEQ ID NO:1 oder SEQ ID NO:2 besteht.

14. Verfahren nach einem der Ansprüche 2 bis 13, wobei:
(i) die Oligonukleotidsonde:
(a) an eine Sequenz bindet, die die Sequenz 5' TAAGCAGTGGGTTCCCT 3' (SEQ ID NO:3) oder eine damit zu wenigstens 90 % identische Sequenz umfasst bzw. daraus besteht; oder
(b) die Sequenz SEQ ID NO:4, SEQ ID NO:5, SEQ ID NO:6, SEQ ID NO:45, SEQ ID NO:46, SEQ ID NO:47 oder SEQ ID NO:48 umfasst bzw. daraus besteht; und
(ii) es sich bei dem Vorwärtsprimer um einen HIV-1-Primer handelt, der aus der Sequenz SEQ ID NO:7 oder SEQ ID NO:29 besteht; und
(iii) es sich bei dem Rückwärtsprimer um einen HIV-1-Primer handelt, der aus der Sequenz SEQ ID NO:9 oder SEQ ID NO:30 besteht.

15. Verfahren nach einem vorhergehenden Anspruch, wobei:
(i) die Oligonukleotidsonde:
(a) an eine Sequenz bindet, die die Sequenz 5' GCCTGGGTGTTCCCTGCTAGACTCT 3' (SEQ ID NO:11) oder eine damit zu wenigstens 90 % identische Sequenz umfasst bzw. daraus besteht; oder
(b) die Sequenz SEQ ID NO:12, SEQ ID NO:13, SEQ ID NO: 37, SEQ ID NO: 38, SEQ ID NO:39 oder SEQ ID NO:40, SEQ ID:41, SEQ ID:42, SEQ ID:43, SEQ ID:44, SEQ ID NO:49, SEQ ID NO:50, SEQ ID NO:51, SEQ ID NO:52 oder SEQ ID NO:53 umfasst bzw. daraus besteht; und
(ii) der HIV-2-Vorwärtsprimer aus der Sequenz gemäß SEQ ID NO:14, SEQ ID NO:33 oder SEQ ID NO:35 besteht; und
(iii) der HIV-2-Rückwärtsprimer aus der Sequenz SEQ ID NO:16, SEQ ID NO:32, SEQ ID NO:34 oder SEQ ID NO:36 besteht.

16. Zusammensetzung, umfassend eine markierte Oligonukleotidsonde sowie einen Vorwärts- und Rückwärtsprimer, jeweils ausgewählt aus einer Kombination von einer/einem oder mehreren des Folgenden:
(i) Oligonukleotidsonde, umfassend oder bestehend aus SEQ ID NO:4, SEQ ID NO:5, SEQ ID NO:6, SEQ ID NO:45, SEQ ID NO:46, SEQ ID NO:47 oder SEQ ID NO:48;
(ii) Vorwärtsprimer, bestehend aus SEQ ID NO:7 oder SEQ ID NO:29; und
(iii) Rückwärtsprimer, bestehend aus SEQ ID NO:9 oder SEQ ID NO:30.

17. Zusammensetzung, umfassend eine markierte Oligonukleotidsonde sowie einen Vorwärts- und Rückwärtsprimer, jeweils ausgewählt aus einer Kombination von einer/einem oder mehreren des Folgenden:
(i) Oligonukleotidsonde, umfassend oder bestehend aus SEQ ID NO:12, SEQ ID NO:13, SEQ ID NO: 37, SEQ ID NO: 38, SEQ ID NO:39, SEQ ID NO:40; SEQ ID NO:41, SEQ ID NO:42, SEQ ID NO:43, SEQ ID NO:44, SEQ ID NO:49, SEQ ID NO:50, SEQ ID NO:51, SEQ ID NO:52 oder SEQ ID NO:53;
(ii) Vorwärtsprimer, bestehend aus SEQ ID NO:14, SEQ ID NO:33 oder SEQ ID NO:35;
(iii) Rückwärtsprimer, bestehend aus SEQ ID NO:16, SEQ ID NO:32, SEQ ID NO:34 oder SEQ ID NO:36.

18. Kit zum Nachweisen von HIV-1, umfassend die die markierte Oligonukleotidsonde, Vorwärts- und Rückwärtsprimer unter Anspruch 16 umfassende Zusammensetzung, zusammen mit geeigneten Reagentien und Anweisungen zum Nachweisen und Quantifizieren von HIV-1.

19. Kit zum Nachweisen von HIV-2, umfassend die die markierte Oligonukleotidsonde, Vorwärts- und Rückwärtsprimer unter Anspruch 17 umfassende Zusammensetzung, zusammen mit geeigneten Reagentien und Anweisungen zum Nachweisen und Quantifizieren von HIV-2.

## Revendications

1. Procédé de détection d'un virus de l'immunodéficience humaine (VIH) chez un sujet atteint de VIH-1 ou VIH-2, ou un sujet suspecté d'être atteint d'une infection par le VIH (SIDA), le procédé comprenant la réalisation d'une amplification PCR d'un acide nucléique de région R dans un échantillon biologique obtenu auprès du sujet, l'amplification comprenant des amorces sens et antisens qui s'hybrident à des séquences situées dans la région R des longues répétitions terminales (LTR) du VIH, et subséquemment la détection d'une quelconque amplification, la détection d'une amplification étant indicative de la présence du VIH chez le sujet, l'amplification entraînera l'amplification des deux régions R présentes dans les LTR 5' et 3', et :
(i) l'amorce sens étant une amorce de VIH-1 constituée de la séquence SEQ ID NO: 7, ou SEQ ID NO: 29 ; et l'amorce antisens étant une amorce de VIH-1 constituée de la séquence SEQ ID NO: 9, ou SEQ ID NO: 30 ; ou
(ii) l'amorce sens étant une amorce de VIH-2 constituée de la séquence SEQ ID NO: 14, SEQ ID NO: 33 ou SEQ ID NO: 35 ; et l'amorce antisens étant une amorce de VIH-2 constituée de la séquence SEQ ID NO: 16, SEQ ID NO: 32, SEQ ID NO: 34 ou SEQ ID NO: 36.

2. Procédé selon la revendication 1, la détection de l'amplification étant réalisée avec une sonde d'oligonucléotide marquée qui s'hybride à une séquence dans la séquence de région R amplifiée.

3. Procédé selon l'une quelconque des revendications 1 à 2, l'acide nucléique étant un ADN ou un ARN transcrit par transcription inverse (ADNc).

4. Procédé selon l'une quelconque des revendications 1 à 3, le procédé comprenant :
(i) la quantification d'un nombre de copies d'ADN de VIH dans un échantillon biologique provenant du sujet atteint de VIH, ou suspecté d'être atteint d'une infection par le VIH, le procédé comprenant la quantification de la séquence de région R-VIH amplifiée par référence à une référence de plasmide de VIH correspondante pour obtenir le nombre de copies d'ADN de VIH par volume d'échantillon ; ou
(ii) la quantification d'un nombre de copies d'ADN de VIH dans un échantillon biologique provenant du sujet atteint de VIH, ou suspecté d'être atteint d'une infection par le VIH, le procédé comprenant la quantification de la séquence de région R-VIH amplifiée par référence à une référence de plasmide de VIH correspondante pour obtenir le nombre de copies d'ARN de VIH par volume d'échantillon.

5. Procédé selon l'une quelconque des revendications 1 à 4, l'amplification étant une PCR en temps réel ou une PCR de point final.

6. Procédé selon la revendication 4 ou 5, comprenant en outre :
(i) la normalisation du nombre de copies d'ADN de VIH par rapport à une référence d'ADN pour obtenir le nombre de copies d'ADN de VIH par cellule(s) dans l'échantillon biologique ; ou
(ii) la normalisation du nombre de copies d'ARN de VIH par rapport à une référence d'ARN pour obtenir le nombre de copies d'ARN de VIH par cellule(s) dans l'échantillon biologique.

7. Procédé selon l'une quelconque des revendications 4 à 6, comprenant :
(i) l'obtention d'une partie aliquote de l'échantillon duquel l'ADN ou l'ARN a été extrait ;
(ii) la mise en contact de la partie aliquote avec une sonde d'oligonucléotide d'hydrolyse marquée qui s'hybride à une séquence de région R d'une longue répétition terminale (LTR) de l'ADN de VIH ou de l'ARN de VIH transcrit par transcription inverse (ADNc) ;
(iii) la mise en contact de la partie aliquote avec des amorces sens et antisens qui s'hybrident à des séquences dans la séquence de région R de l'ADN de VIH ou de l'ARN de VIH transcrit par transcription inverse (ADNc) ;
(iv) l'amplification de la séquence de région R par PCR ;
(v) l'extrapolation du signal obtenu auprès de l'oligonucléotide marqué à une courbe de référence obtenue par des amplifications correspondantes de dilutions en série d'une référence de VIH pour calculer le nombre de copies d'ADN ou d'ARN de région R de VIH par volume d'ADN ou d'ARN dans la partie aliquote.

8. Procédé selon l'une quelconque des revendications 1 à 4, l'amorce sens ou antisens étant marquée par de la biotine et la sonde étant marquée par de la digoxigénine (Dig).

9. Procédé selon l'une quelconque des revendications 1 à 8,
(i) l'amorce sens étant la SEQ ID NO: 7 et l'amorce antisens étant la SEQ ID NO: 9 ;
(ii) l'amorce sens étant la SEQ ID NO: 14 et l'amorce antisens étant la SEQ ID NO: 16 ;
(iii) l'amorce sens étant la SEQ ID NO: 33 et l'amorce antisens étant la SEQ ID NO: 34 ;
(iv) l'amorce sens étant la SEQ ID NO: 35 et l'amorce antisens étant la SEQ ID NO: 36.

10. Procédé pour surveiller une thérapie antirétrovirale (ART) qui est administrée à un sujet positif au VIH, comprenant la quantification du nombre de copies d'ADN de VIH selon l'une quelconque des revendications 4 à 8 sur au moins deux points dans le temps et la comparaison de la différence du nombre de copies d'ADN de région R de VIH entre les au moins deux points dans le temps, une augmentation du nombre de copies d'ADN de région R de VIH indiquant que le sujet reçoit une ART optimale/efficace.

11. Procédé pour l'évaluation de l'efficacité d'une thérapie antirétrovirale (ART) administrée à un sujet positif au VIH, le procédé comprenant :
(i) la quantification du nombre de copies d'ADN de région R de VIH selon l'une quelconque des revendications 4 à 7 ;
(ii) la quantification du nombre de copies d'ARN de région R de VIH selon l'une quelconque des revendications 4 à 7 ;
(iii) la détermination d'un nombre de copies d'ARN de VIH normalisé dans l'échantillon en divisant la valeur obtenue dans l'étape (i) par celle obtenue dans l'étape (ii) ; et
(iv) la comparaison de la valeur du nombre de copies d'ARN de VIH normalisé avec une ou plusieurs valeurs normalisées précédentes obtenues auprès du même sujet ;
une diminution du nombre de copies d'ARN de VIH normalisé indiquant que le sujet reçoit une ART optimale/efficace.

12. Procédé selon la revendication 11 comprenant en outre la préparation d'ADN et d'ARN d'un échantillon biologique qui a été obtenu auprès du sujet, l'échantillon biologique étant une population de cellules choisies parmi le sang ou un tissu ou un quelconque autre fluide biologique dans lequel des cellules infectées par le VIH sont présentes, par exemple l'échantillon biologique étant des PBMC.

13. Procédé selon une quelconque revendication précédente, la séquence de région R étant constituée de la séquence indiquée dans la SEQ ID NO: 1 ou la SEQ ID NO: 2.

14. Procédé selon l'une quelconque des revendications 2 à 13,
(i) la sonde d'oligonucléotide :
(a) se liant à une séquence comprenant ou constituée de la séquence 5' **TAAGCAGTGGGTTCCCT 3' (SEQ ID NO:3)** ou d'une séquence au moins 90 % identique à celle-ci ; ou
(b) comprenant ou étant constituée de la séquence SEQ ID NO: 4, SEQ ID NO: 5, SEQ ID NO: 6, SEQ ID NO: 45, SEQ ID NO: 46, SEQ ID NO: 47, ou SEQ ID NO: 48 ; et
(ii) l'amorce sens étant une amorce de VIH-1 constituée de la SEQ ID NO: 7, ou de la SEQ ID NO: 29 ; et
(iii) l'amorce antisens étant une amorce de VIH-1 constituée de la SEQ ID NO: 9, ou de la SEQ ID NO: 30.

15. Procédé selon une quelconque revendication précédente,
(i) la sonde d'oligonucléotide :
(a) se liant à une séquence comprenant ou constituée de la séquence 5' **GCCTGGGTGTTCCCTGCTAGACTCT 3'** (SEQ ID NO: 11) ou une séquence au moins 90 % identique à celle-ci ; ou
(b) comprenant ou étant constituée de la séquence SEQ ID NO: 12, SEQ ID NO: 13, SEQ ID NO: 37, SEQ ID NO: 38, SEQ ID NO: 39, ou SEQ ID NO: 40, SEQ ID NO: 41, SEQ ID NO: 42, SEQ ID NO: 43, SEQ ID NO: 44, SEQ ID NO: 49, SEQ ID NO: 50, SEQ ID NO: 51, SEQ ID NO: 52, ou SEQ ID NO: 53 ; et
(ii) l'amorce sens de VIH-2 étant constituée de la séquence selon la SEQ ID NO: 14, SEQ ID NO: 33, ou SEQ ID NO: 35 ; et
(iii) l'amorce antisens de VIH-2 étant constituée de la séquence SEQ ID NO: 16, SEQ ID NO: 32, SEQ ID NO: 34 ou SEQ ID NO: 36.

16. Composition comprenant une sonde d'oligonucléotide marquée et une amorce sens et antisens choisie parmi une combinaison d'une ou plusieurs de ce qui suit :
(i) une sonde d'oligonucléotide comprenant ou constituée de la SEQ ID NO: 4, SEQ ID NO: 5, SEQ ID NO: 6, SEQ ID NO: 45, SEQ ID NO: 46, SEQ ID NO: 47, ou SEQ ID NO: 48 ;
(ii) une amorce sens constituée de la SEQ ID NO: 7 ou SEQ ID NO: 29 ; et
(iii) une amorce antisens constituée de la SEQ ID NO: 9 ou la SEQ ID NO: 30.

17. Composition comprenant une sonde d'oligonucléotide marquée et une amorce sens et antisens choisie parmi une combinaison d'une ou plusieurs de ce qui suit :
(i) une sonde d'oligonucléotide comprenant ou constituée de la SEQ ID NO: 12, SEQ ID NO: 13, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 49, SEQ ID NO: 50, SEQ ID NO: 51, SEQ ID NO: 52, ou SEQ ID NO: 53 ;
(ii) une amorce sens constituée de la SEQ ID NO: 14, SEQ ID NO: 33 ou SEQ ID NO: 35 ; et
(iii) une amorce antisens constituée de la SEQ ID NO: 16, SEQ ID NO: 32, SEQ ID NO: 34 ou SEQ ID NO: 36.

18. Kit pour la détection du VIH-1, comprenant la composition comprenant la sonde d'oligonucléotide marquée, une amorce sens et antisens selon la revendication 16, conjointement avec des réactifs appropriés et des instructions pour la détection et la quantification du VIH-1.

19. Kit pour la détection du VIH-2, comprenant la composition comprenant la sonde d'oligonucléotide marquée, une amorce sens et antisens selon la revendication 17, conjointement avec des réactifs appropriés et des instructions pour la détection et la quantification du VIH-2.
